# EUROPEAN PATENT APPLICATION

(11) **EP 4 335 456 A1**
(43) Date of publication of application: **13.03.2024**
(21) Application number: 22806613.0
(22) Date of filing: 06.05.2022
(51) Int. Cl.: A61K 39/395, C07K 16/00

(54) **BINDING MOLECULE AGAINST DLL3 AND USE THEREOF**

(30) Priority: 08.05.2021 CN 202110499247; 24.04.2022 CN 202210460557
(71) Applicant: Shanghai Qilu Pharmaceutical Research and Development Centre Ltd., Shanghai 201203 (CN)
(72) Inventor: YANG, Liuqing, Shanghai 201203 (CN); LI, Ruimei, Shanghai 201203 (CN); GU, Jinming, Shanghai 201203 (CN); CHOU, Chuan-Chu, Shanghai 201203 (CN)
(74) Representative: Elzaburu S.L.P.
(86) International application number: PCT/CN2022/091238
(87) International publication number: WO 2022/237647

(57) **Abstract**

A binding molecule against DLL3 or an antigen-binding fragment thereof, a derivative containing the binding molecule or the antigen-binding fragment thereof, and a pharmaceutical composition. In addition, the present invention also relates to the related use of the binding molecule or the antigen-binding fragment thereof in the treatment of cancers and in detection and diagnosis.

## Description

### FIELD OF THE INVENTION

The present disclosure belongs to the field of immunology, in particular, the present disclosure relates to a binding molecule against DLL3 or an antigen-binding fragment thereof, a derivative containing the binding molecule or the antigen-binding fragment thereof, and a pharmaceutical composition, and related application thereof in the treatment of cancer.

### BACKGROUND TO THE INVENTION

Lung cancer is one of the malignant tumors with the fastest increase in morbidity and mortality and the greatest threat to human health and life. According to pathology, it is divided into small cell lung cancer and non-small cell lung cancer.

Non-small cell lung cancer (NSCLC) is the most important form of lung cancer, and more than 80% of lung cancer patients are of this type. Targeted therapy of lung cancer, PD-1 immunodrugs, and gene sequencing nowadays receive attention, and the following breakthrough progress is mainly focused on non-small cell lung cancer, which greatly increases the available and effective treatment of this subgroup of lung cancer, and greatly improves the five-year survival rate of non-small cell lung cancer patients.

Small cell lung cancer (SCLC) is a highly aggressive, fatal, and widely metastatic lung cancer, accounting for about 15% of lung cancers, and is very different from other lung cancers in pathology, molecular biology, biology, and clinical. It is estimated that over 234, 000 SCLC patients are diagnosed each year, resulting in approximately 250, 000 deaths worldwide each year. SCLC is characterized in rapid tumor growth, high vascularity, unstable genome, and early metastatic spread. Only modest improvements in SCLC detection, treatment, or survival have been seen over the past 30 years, leading to the classification of SCLC as a refractory cancer. Local treatment, such as surgery or radiotherapy, or a combination of both, is almost impossible to cure completely. Platinum-based combination chemotherapy remains the cornerstone of treatment. The first-line standard regimen consists of platinum (carboplatin or cisplatin) in combination with other cytotoxic drugs (such as etoposide). The response rate of chemotherapy for small cell lung cancer is very high, but it is also often accompanied by recurrence, especially in patients with the extensive stage. For patients with limited stage, the median survival period is 14-20 months, while for patients with extensive stage, it is only 9-11 months. For patients with recurrence, the survival period is shorter and there is almost no treatment option. Topotecan, the only one recommended by FDA, is limited for its Hematology toxicity, and the treatment response rate is also unsatisfactory, only 5-24%. The median survival time is less than 25 weeks. Currently, there is no specific third-line treatment recommendation, so a new effective therapeutic drug is urgently needed.

Delta-like 3, also known as DLL3, is a protein encoded by DLL3 gene and is one of the ligands of the Notch family. DLL3 has only 36% homology with DLL1, and unlike other delta type dsl (delta/serate/lag-2) proteins, such as DLL1 and DLL4, DLL3 has the highest expression in normal tissues of a fetal brain and plays a key role in the growth and development of paraxial mesoderm. It was found that it was expressed on the surface of tumor cells in about 85% of patients with small cell lung cancer and large cell neuroendocrine cancer, and also highly expressed in glioblastoma multiforme, melanoma, pancreatic cancer, and rectal cancer. But it is not expressed in healthy tissues and non-neuroendocrine tumors. This protein is involved in influencing the Notch regulatory signaling pathway such that signaling from the Notch pathway ultimately promotes unrestricted growth of cancer. In normal tissues, mRNA expression of DLL3 is restricted to the brain, esophagus, and pancreas.

Studies have further shown that by detecting the expression of DLL3 in whole transcriptome sequencing data of primary SCLC biopsies, SCLC cell lines, and normal lung biopsies in the analysis of tumor tissue and normal tissue specimens, it was shown that the mRNA of DLL3 in SCLC is increased about 35-fold relative to a normal lung. These SCLC tumor samples were compared with transcriptome data from normal tissues and other tumor types in the cancer genome to further confirm that DLL3 expression in primary SCLC tumor samples and low-grade gliomas (LGG), glioblastoma (GBM), and melanoma (SKCM) was increased. Illumina BeadChip data of the clinical lung cancer genome project also showed elevated DLL3 in primary SCLC tumor specimens compared to NSCLC.

Data obtained from Cancer Cell Line Encyclopedia further confirmed that expression of DLL3 mRNA is particularly elevated in the SCLC cell line. Collectively, these expression data across multiple technology platforms and samples suggest that DLL3 mRNA is overexpressed in primary SCLC tumors, SCLC PDX, traditional SCLC cell lines, and LCNEC PDX, whereas mRNA expression in normal tissues is primarily restricted to the brain.

### SUMMARY

The object of the present disclosure provides a binding molecule against DLL3 and an antigen-binding fragment thereof, the binding molecule or antigen-binding fragments thereof being capable of specifically binding to DLL3 without non-specifically binding to the family proteins DLL1 and DLL4.

A first aspect of the present disclosure provides a DLL3 binding molecule or an antigen binding fragment thereof, the DLL3 binding molecule or the antigen binding fragment thereof including:
i) a heavy chain complementary determinant region 1 (HCDR1) selected from SEQ ID NOs: 6, 9, 12, 15, or 18;
ii) a heavy chain complementary determinant region 2 (HCDR2) selected from: SEQ ID NOs: 7, 10, 13, 16, or 19; and
iii) a heavy chain complementary determinant region 3 (HCDR3) selected from: SEQ ID NOs: 8, 11, 14, 17, 20, 110, or 111.

In an embodiment, the DLL3 binding molecule or the antigen binding fragment thereof comprises a heavy chain variable region (VH), the heavy chain variable region comprises HCDR1, HCDR2, and HCDR3, and the amino acid sequences of the HCDR1, HCDR2, and HCDR3 are respectively:
a) SEQ ID NOs: 6, 7, and 8; or
b) SEQ ID NOs: 9, 10, and 11; or
c) SEQ ID NOs: 12, 13, and 14; or
d) SEQ ID NOs: 15, 16, and 17; or
e) SEQ ID NOs: 18, 19, and 20; or
f) SEQ ID NOs: 6, 7, and 110; or
g) SEQ ID NOs: 6, 7, and 111.

In an embodiment, the heavy chain variable region comprises an amino acid sequence selected from any one of SEQ ID NOs: 1-5, 21-29, or comprises an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to any one of SEQ ID NOs: 1-5, 21-29.

A second aspect of the present disclosure provides another DLL3 binding molecule or an antigen binding fragment thereof, the DLL3 binding molecule or the antigen binding fragment thereof including:
i) a heavy chain complementary determinant region 1 (HCDR1) selected from: SEQ ID NOs: 57, 63, 69, 72, 78, 84, 93, or 99.
ii) a heavy chain complementary determinant region 2 (HCDR2) selected from: SEQ ID NOs: 58, 64, 70, 73, 79, 85, 94, or 100.
iii) a heavy chain complementary determinant region 3 (HCDR3) selected from: SEQ ID NOs: 59, 65, 71, 74, 80, 86, 95, or 101.
iv) a light chain complementary determinant region 1 (LCDR1) selected from: SEQ ID NOs: 60, 66, 75, 81, 87, 90, 96, or 102.
v) a light chain complementary determinant region 2 (LCDR2) selected from: SEQ ID NOs: 61, 67, 76, 82, 88, 91, 97, or 103; and
vi) a light chain complementary determinant region 3 (LCDR3) selected from: SEQ ID NOs: 62, 68, 77, 83, 89, 92, 98, 104, 112, 113, or 114.

In an embodiment, the DLL3 binding molecule or the antigen binding fragment thereof comprises a heavy chain variable region (VH) including HCDR1, HCDR2, and HCDR3 and a light chain variable region (VL) including LCDR1, LCDR2, and LCDR3, wherein the amino acid sequences of the HCDR1, HCDR2, HCDR3, LCDR1, LCDR2, and LCDR3 are respectively:
a) SEQ ID NOs: 57, 58, 59, 60, 61, and 62; or
b) SEQ ID NOs: 63, 64, 65, 66, 67, and 68; or
c) SEQ ID NOs: 69, 70, 71, 66, 67, and 68; or
d) SEQ ID NOs: 72, 73, 74, 75, 76 and 77; or
e) SEQ ID NOs: 78, 79, 80, 81, 82, and 83; or
f) SEQ ID NOs: 84, 85, 86, 87, 88, and 89; or
g) SEQ ID NOs: 84, 85, 86, 90, 91 and 92; or
h) SEQ ID NOs: 93, 94, 95, 96, 97, and 98; or
i) SEQ ID NOs: 99, 100, 101, 75, 76 and 77; or
j) SEQ ID NOs: 72, 73, 74, 102, 103 and 104; or
k) SEQ ID NOs: 63, 64, 65, 66, 67, and 112; or
l) SEQ ID NOs: 63, 64, 65, 66, 67, and 113; or
m) SEQ ID NOs: 63, 64, 65, 66, 67, and 114.

In an embodiment, the heavy chain variable region comprises an amino acid sequence selected from any one of SEQ ID NOs: 30, 32, 34, 35, 37, 39, 42, 44, 46, 49, or 51, or comprises an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to any one of SEQ ID NOs: 30, 32, 34, 35, 37, 39, 42, 44, 46, 49, or 51.

In an embodiment, the light chain variable region comprises an amino acid sequence selected from any one of SEQ ID NOs: 31, 33, 36, 38, 40, 41, 43, 45, 47, 48, 50, 52, 53, 54, 55, or 56, or comprises an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to any one of SEQ ID NOs: 31, 33, 36, 38, 40, 41, 43, 45, 47, 48, 50, 52, 53, 54, 55, or 56.

In an embodiment, the heavy chain variable region and the light chain variable region each comprise sequences selected from a group consisting of:
1) SEQ ID NOs: 30 and 31; or
2) SEQ ID NOs: 32 and 33; or
3) SEQ ID NOs: 34 and 33; or
4) SEQ ID NOs: 35 and 36; or
5) SEQ ID NOs: 37 and 38; or
6) SEQ ID NOs: 39 and 40; or
7) SEQ ID NOs: 39 and 41; or
8) SEQ ID NOs: 42 and 43; or
9) SEQ ID NOs: 44 and 36; or
10) SEQ ID NOs: 35 and 45; or
11) SEQ ID NOs: 46 and 47; or
12) SEQ ID NOs: 46 and 48; or
13) SEQ ID NOs: 49 and 50; or
14) SEQ ID NOs: 51 and 52; or
15) SEQ ID NOs: 51 and 53; or
16) SEQ ID NOs: 46 and 54; or
17) SEQ ID NOs: 46 and 55; or
18) SEQ ID NOs: 46 and 56.

The DLL3 binding molecule or antigen binding fragment thereof of the present disclosure further comprises a heavy chain constant region and/or a light chain constant region; preferably, the heavy chain constant region comprises an Fc; more preferably, Fc is derived from murine or human; more preferably, the sequence of the Fc is native or modified. The DLL3 binding molecules or antigen binding fragment thereof of the present disclosure can be a monoclonal antibody, a bispecific binding molecule, a multispecific binding molecule, a humanized antibody, a chimeric antibody, a modified antibody, a fully human antibody, a full-length antibody, a heavy chain antibody, a nanobody, an Fab, an Fv, an scFv, an F(ab')2, a linear antibody, or a single domain antibody.

The DLL3 binding molecules or antigen binding fragments thereof of the present disclosure may be in the form of IgG1, IgG2, IgG3, or IgG4.

The present disclosure also provides a conjugate formed by coupling the DLL3 binding molecule or the antigen binding fragment thereof of the present disclosure to a capture label or a detection label; preferably, the detection label comprises a radionuclide, a luminescent substance, a colored substance, or an enzyme.

The present disclosure also provides an antibody drug conjugate (ADC) formed by coupling the DLL3 binding molecule or the antigen binding fragment thereof of the present disclosure with another biologically active molecule; the other biologically active molecule is a small molecule drug; preferably, the DLL3 binding molecule or the antigen binding fragment thereof is linked to the other biologically active molecule via a linker.

The disclosure also provides a nucleic acid encoding the DLL3 binding molecule or the antigen binding fragment thereof of the present disclosure, as well as a recombinant vector including the nucleic acid, and a host cell including the nucleic acid or vector described above. Preferably, the host cell is a prokaryotic cell, preferably E. coli, or a eukaryotic cell, preferably a mammalian cell or yeast; further preferably, the mammalian cell is a CHO cell or a HEK293 cell.

The present disclosure also provides a method for preparing the DLL3 binding molecule or the antigen binding fragment thereof of the present disclosure, the method including: culturing the host cell described above under suitable conditions and purifying an expression product from the cell.

The present disclosure also provides the use of the DLL3 binding molecule or the antigen binding fragment thereof of the present disclosure in the manufacture of a medicament for the treatment or amelioration of a tumor.

In an embodiment, the medicament targets a tumor cell aberrantly expressing DLL3.

In an embodiment, the tumor is selected from: small cell lung cancer, glioblastoma, neuroendocrine cancer, melanoma, pancreatic cancer, rectal cancer, and metastatic cancers of the aforementioned tumors.

The present disclosure also provides the use of the DLL3 binding molecule or the antigen binding fragment thereof of the present disclosure in the manufacture of a medicament for the treatment or amelioration of a tumor.

In an embodiment, the detection reagent is used for detecting expression of DLL3; the diagnostic reagent is used for diagnosing a tumor; preferably, the tumor is selected from: small cell lung cancer, glioblastoma, neuroendocrine cancer, melanoma, pancreatic cancer, rectal cancer, and metastatic cancers of the aforementioned tumors.

The present disclosure also provides a method for detecting DLL3 expression in a sample, the method including:
(1) contacting the sample with the DLL3 binding molecule or the antigen binding fragment thereof of the present disclosure; and
(2) detecting the formation of a complex of the DLL3 binding molecule or the antigen binding fragment thereof and DLL3; optionally, the DLL3 binding molecule or the antigen binding fragment thereof is detectably labeled.

The present disclosure also provides a pharmaceutical composition including an effective amount of the DLL3 binding molecules or the antigen binding fragment thereof of the present disclosure, or an effective amount of the antibody drug conjugates of the present disclosure, or an effective amount of the nucleic acid of the present disclosure, or an effective amount of the recombinant vector of the present disclosure, or an effective amount of the host cell of the present disclosure.

In an embodiment, the pharmaceutical composition also comprises a pharmaceutically acceptable carrier.

Preferably, the pharmaceutical composition further comprises one or more additional therapeutic agents.

The present disclosure also provides a drug box or kit including a container and the pharmaceutical composition of the present disclosure in the container.

The present disclosure also provides a method for inducing death of a cell expressing DLL3, the method including contacting the cell with the pharmaceutical composition of the present disclosure, the cell expressing DLL3 being a tumor cell.

In an embodiment, the tumor cell is a cell selected from the following tumors: small cell lung cancer, glioblastoma, neuroendocrine cancer, melanoma, pancreatic cancer, rectal cancer, and metastatic cancers of the aforementioned tumors.

The present disclosure also provides a method for treating a disease associated with the expression of DLL3 in a subject, the method including administering the pharmaceutical composition of the present disclosure, or the drug box or kit of the foregoing to a subject in need thereof.

In an embodiment, the disease is a tumor; small cell lung cancer, glioblastoma, neuroendocrine cancer, melanoma, pancreatic cancer, rectal cancer, and metastatic cancer of the aforementioned tumors.

In an embodiment, the method further comprises administering an additional therapeutic agent to the subject.

The technical solutions of the present disclosure have the following advantageous effects: the DLL3 binding molecule and the antigen binding fragment thereof of the present disclosure is capable of specifically binding to DLL3 without non-specifically binding to the family proteins DLL1 and DLL4.

### BRIEF DESCRIPTION OF THE FIGURES

The figures further illustrate the novel features disclosed herein. The features and advantages disclosed in this specification will be better understood with reference to the figures, but it is understood that the figures are merely for purposes of illustrating specific embodiments of the principles disclosed herein and are not intended to limit the scope of the appended claims.
FIG. 1A shows the binding of camelid-derived anti-DLL3 chimeric antibodies of the present disclosure to DLL3-expressing cells (SHP-77).
FIG. 1B shows the binding of camelid-derived anti-DLL3 chimeric antibodies of the present disclosure to DLL3-expressing cells (HEK293-cyno DLL3).
FIG. 2A shows the binding of camelid-derived humanized anti-DLL3 antibodies of the present disclosure to DLL3-expressing cells (SHP-77).
FIG. 2B shows the binding of camelid-derived humanized anti-DLL3 antibodies of the present disclosure to DLL3-expressing cells (HEK293-cyno DLL3).
FIG. 3A shows the binding of camelid-derived humanized anti-DLL3 antibodies of the present disclosure to the family protein DLL1.
FIG. 3B shows the binding of camelid-derived humanized anti-DLL3 antibodies of the present disclosure to the family protein DLL4.
FIG. 4 shows the binding of camelid-derived hDLL3-3-1 humanized antibody variants of the present disclosure to DLL3-expressing cells (SHP-77).
FIG. 5A shows binding of mouse hybridoma-derived anti-DLL3 chimeric antibodies of the present disclosure to DLL3-expressing cells (SHP-77).
FIG. 5B shows binding of mouse hybridoma-derived anti-DLL3 chimeric antibodies of the present disclosure to DLL3-expressing cells (HEK293-cyno DLL3).
FIG. 6 shows the binding of mouse hybridoma-derived humanized anti-DLL3 antibodies of the present disclosure to DLL3-expressing cells (SHP-77).
FIG. 7A shows the binding of mouse hybridoma-derived humanized anti-DLL3 antibodies of the present disclosure to the family protein DLL1.
FIG. 7B shows the binding of mouse hybridoma-derived humanized anti-DLL3 antibodies of the present disclosure to the family protein DLL4.

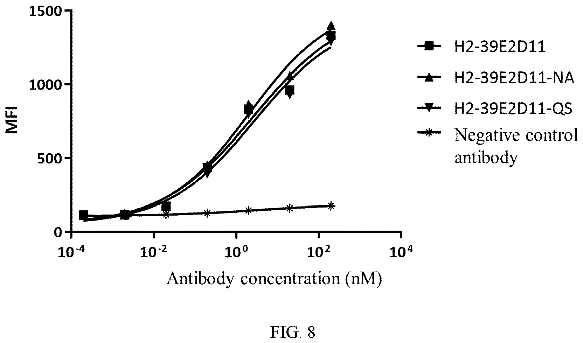

FIG. 8 shows the binding of mouse hybridoma-derived humanized H2-39E2D11 antibody variants of the present disclosure to DLL3-expressing cells (SHP-77).

### DETAILED DESCRIPTION OF THE INVENTION

### TERMS

All publications, patents, and patent applications mentioned in this specification are herein incorporated by reference to the same extent as if each publication, patent, or patent application was specifically and individually indicated to be incorporated by reference.

Before the present disclosure is described in detail below, it is to be understood that the present disclosure is not limited to the particular methodology, protocols, and reagents described herein, as these may vary. It should also be understood that the terms used herein are only intended to describe specific embodiments and are not intended to limit the scope of the present disclosure. Unless otherwise defined, all technical and scientific terms used herein have the same meaning as commonly understood by one of ordinary skill in the art to which the present disclosure belongs.

Certain embodiments disclosed herein encompass numerical ranges, and certain aspects of the present disclosure may be described in terms of ranges. Unless otherwise indicated, it is to be understood that the numerical ranges or descriptions of ranges are merely for brevity and convenience and are not to be construed as strictly limiting the scope of the present disclosure. Therefore, the description using a range approach should be considered as specifically disclosing all possible sub-ranges and all possible specific numerical points within that range, as these sub-ranges and numerical points have been clearly stated herein. The above principles apply equally regardless of the breadth of the numerical values recited. When a range description is used, the range includes the endpoints of the range.

The term "about", when referring to a measurable value such as an amount, temporal duration, and the like, is meant to encompass variations of ± 20%, or in some cases ± 10%, or in some cases ± 5%, or in some cases ± 1%, or in some cases ± 0.1% of the specified value.

The three-letter code and the one-letter code for amino acids used herein are as described in J. Biol. Chem, 243, p3558 (1968).

As used herein, the term "antibody" may include intact antibodies (e.g. full-length monoclonal antibodies) and any antigen-binding fragment (i.e. antigen-binding portion) thereof or single chain thereof, as well as a product having antigen-specific binding capability that is engineered (e.g. linked to other peptide segments, rearranged functional units, etc.) based on the intact antibody or antigen-binding fragment or the single chain thereof.

In an embodiment, an antibody typically refers to a Y-type tetrameric protein comprising two heavy (H) polypeptide chains and two light (L) polypeptide chains held together by covalent disulfide bonds and non-covalent interactions. The native IgG antibody has such a structure. Each light chain consists of a variable domain (VL) and a constant domain (CL). Each heavy chain consists of a variable domain (VH) and a constant region.

There are five main categories of antibodies known in the art: IgA, IgD, IgE, IgG, and IgM, with corresponding heavy chain constant domains called α, δ, ε, γ and µ; IgG and IgA can be further divided into different subclasses, for example, IgG can be divided into IgG1, IgG2, IgG3, IgG4; IgA can be divided into IgA1 and IgA2. The light chain of antibodies from any vertebrate species can be assigned to one of two distinct types based on their constant domain amino acid sequence, called κ and λ_{∘}

In the case of the IgG, IgA, and IgD antibodies, the constant region comprises three domains designated CH1, CH2, and CH3 (IgM and IgE have a fourth domain CH4). In the IgG, IgA, and IgD classes, the CH1 and CH2 domains are separated by a flexible hinge region that is a variable-length proline and cysteine-rich segment. Each class of antibodies further comprises inter-and intra-chain disulfide bonds formed from the paired cysteine residues.

The term "variable region" or "variable domain" shows a significant change in amino acid composition from one antibody to another and is primarily responsible for antigen recognition and binding. The variable region of each light/heavy chain pair forms the antibody binding site such that the intact IgG antibody has two binding sites (i.e. it is bivalent). The variable region (VH) of the heavy chain and the variable region (VL) of the light chain each comprise three regions of extreme variability, referred to as HVR or, more generally, as complementary determinant region (CDR), each VH and VL having four framework regions FR, designated FR1, FR2, FR3, FR4, respectively. Thus, the CDR and FR sequences typically appear in the heavy chain variable domain (or light chain variable domain) in the following sequences: FR1-HCDR1(LCDR1)-FR2-HCDR2(LCDR2)-FR3-HCDR3(LCDR3)-FR4.

The term "Fc" is used to define the C-terminal region of an immunoglobulin heavy chain comprising at least a portion of a constant region. This term includes the natural sequence Fc region and the variant Fc region.

As used herein, "antibody" is used in the broadest sense and may include, for example, polyclonal antibodies, monoclonal antibodies, chimeric antibodies, humanized and primatized antibodies, CDR-grafted antibodies, human antibodies (including recombinant human antibodies), recombinant antibodies, intracellular antibodies, multispecific antibodies, bispecific antibodies, monovalent antibodies, multivalent antibodies, anti-idiotypic antibodies, synthetic antibodies (including muteins and variants), and the like. The term "monoclonal antibody" (or "mAb ") refers to an antibody produced by a single cell clone that is substantially homogeneous and directed only to a particular epitope. Monoclonal antibodies can be prepared using a variety of techniques known in the art, including hybridoma techniques, recombinant techniques, phage display techniques, transgenic animals, synthetic techniques, combinations thereof, and the like.

Note that the partitioning of CDR and FR for the variable regions of the monoclonal antibodies of the present disclosure is determined according to the Kabat definition. Other naming and numbering systems, such as Chothia, IMGT, or AHo, are known to those skilled in the art. Thus, humanized antibodies comprising one or more CDR derived from any nomenclature based on the sequences of the monoclonal antibodies of the present disclosure clearly remain within the scope of the present disclosure.

The term "humanized antibody" refers to an antibody in which all or a portion of the amino acids other than CDR of a non-human antibody (e.g. a mouse antibody) have been replaced with the corresponding amino acids derived from a human immunoglobulin. Small additions, deletions, insertions, substitutions, or modifications of amino acids are permissible so long as they do not eliminate the ability of the antibody to bind a particular antigen. A "humanized" antibody retains similar antigen specificity as the original antibody.

The term "chimeric antibody" refers to an antibody in which the variable region originates from one specie and the constant region originates from another specie, e.g., an antibody in which the variable region originates from a mouse antibody and the constant region originates from a human antibody.

The term "antibody fragment" encompasses at least a portion of an intact antibody. As used herein, a "fragment" of an antibody molecule includes an "antigen-binding fragment" of an antibody, and the term "antigen-binding fragment" refers to a polypeptide fragment of an immunoglobulin or antibody that specifically binds to or reacts with a selected antigen or immunogenic determining portion thereof, or a fusion protein product further derived from the fragment, e.g. a single chain antibody, an extracellular binding region in a chimeric antigen receptor, etc. Exemplary antibody fragments or antigen-binding fragments thereof include but are not limited to variable light chain fragments, variable heavy chain fragments, Fab fragments, F(ab')2 fragments, Fd fragments, Fv fragments, single domain antibodies, linear antibodies, single chain antibodies (scFv), bispecific or multispecific antibodies formed from antibody fragments, and the like.

The term "antigen" refers to a substance that is recognized and specifically bound by an antibody or antibody binding fragment. In a broad sense, an antigen can include any immunogenic fragment or determinant of a selected target, including a single epitope, multiple epitopes, single domain, multiple domain, complete extracellular ECD, or protein. Peptides, proteins, glycoproteins, polysaccharides, lipids, portions thereof, and combinations thereof may constitute antigens. Non-limiting exemplary antigens include tumor antigens or pathogen antigens and the like. An "antigen" may also refer to a molecule that elicits an immune response. Any form of antigen or cell or preparation containing the antigen may be used to generate an antibody specific for an antigenic determinant. The antigen can be an isolated full-length protein, a cell surface protein (e.g. immunized with the cell expressing at least a portion of the antigen on its surface), or a soluble protein (e.g. immunized with only the ECD portion of the protein) or a protein construct (e.g. an Fc antigen). The antigen may be produced in a genetically modified cell. Any of the foregoing antigens may be used alone or in combination with one or more immunogenicity enhancing adjuvants known in the art. The DNA encoding the antigen may be genomic or non-genomic (e.g. cDNA) and may encode at least a portion of the ECD sufficient to elicit an immunogenic response. Any vector can be used to transform cells in which the antigen is expressed, including, but not limited to, adenoviral vectors, lentiviral vectors, plasmids, and non-viral vectors such as cationic lipids.

The term "epitope" refers to a site on an antigen to which an immunoglobulin or antibody specifically binds. Epitopes can be formed by adjacent amino acids or non-adjacent amino acids that are juxtaposed through the tertiary folding of proteins. Epitopes formed by adjacent amino acids are usually maintained after exposure to denaturing solvents, while epitopes formed through tertiary folding are typically lost after treatment with denaturing solvents. Epitopes typically exist in a unique spatial conformation and comprise at least 3-15 amino acids. Methods for determining the epitope to which a given antibody binds are well-known in the art and include immunoblotting and immunoprecipitation assays, among others. The methods for determining the spatial conformation of the epitope include techniques in this field, such as X-ray crystal analysis and two-dimensional nuclear magnetic resonance.

The terms "bispecific binding molecule", and "multispecific binding molecule" refer to a binding molecule (e.g. an antibody or a molecule comprising an antibody fragment), preferably a bispecific antibody, having specificity for two or more different antigens (or epitopes), respectively.

When using the variable region in the present disclosure to produce antibodies, binding molecules, bispecific binding molecules, or multispecific binding molecules, the constant region is not particularly limited. It is possible to use a well-known constant region or a self-obtained constant region by those skilled in the art and to introduce amino acid mutations (such as mutations increasing or decreasing bonding to Fcy receptor or FcRn) in the constant region part.

The method for obtaining the binding molecules, antigen-binding fragments, antibodies, bispecific binding molecules, or multispecific binding molecules of the present disclosure is not particularly limited and may be obtained by any method, e.g. Cold Spring Harbor's Using Antibodies: A Laboratory Manual, chapters 5-8 and 15. The binding molecules, antigen-binding fragments, antibodies, bispecific binding molecules, or multi-specific binding molecules invented can be prepared and purified using conventional methods. For example, cDNA sequences encoding heavy and light chains can be cloned and recombined into expression vectors. Recombinant immunoglobulin expression vectors can be stably transfected into CHO cells. As a more recommended existing technique, mammalian expression systems can lead to glycosylation of antibodies, especially in the highly conserved N-terminus of the Fc region. Stable clones are obtained by expressing antibodies that specifically bind to human antigens. Positive clones were expanded in a serum-free medium in a bioreactor to produce antibodies. The antibody-secreting medium can be purified and collected using conventional techniques. The antibodies may be concentrated by filtration using conventional methods. Soluble mixtures and multimers may also be removed by conventional methods, such as molecular sieves, and ion exchange.

The term "antibody drug conjugate" (ADC) refers to an antibody that has covalently conjugated to a therapeutic active substance or active pharmaceutical ingredient (API), so that the therapeutic active substance or active pharmaceutical ingredient (API) can target the binding target of the antibody to demonstrate its pharmacological function. The therapeutically active substance or active pharmaceutical ingredient may be a cytotoxin capable of killing cells targeted by ADC, preferably malignant or cancer cells. The covalent bonding of therapeutic active substances, active pharmaceutical ingredients, or cytotoxics can be performed in a non-site specific manner using standard chemical linkers that couple payloads to lysine or cysteine residues, or preferably, the conjugation is performed in a site-specific manner, which allows full control of the conjugation site and the ratio of drug specific antibodies to the generated ADC.

The term "affinity" or "binding affinity" refers to the strength of all non-covalent interactions between a single binding site of a molecule (such as an antibody) and its binding partner (such as an antigen). The term "KD" refers to the dissociation constant of a particular antibody-antigen interaction. Binding affinity can be determined using various techniques known in the art, such as surface plasmon resonance, biolayer interferometry, dual polarization interferometry, static light scattering, dynamic light scattering, isothermal titration calorimetry, ELISA, analytical ultracentrifugation, and flow cytometry, among others.

The term "biological activity" refers to the ability of an antibody to bind an antigen and cause a measurable biological response, which can be measured in vitro or in vivo.

The pharmaceutical compositions of the present disclosure can be formulated in admixture with suitable pharmaceutically acceptable carriers, vehicles, and the like which are inert, as desired, for example, physiological saline, sterile water, excipients, stabilizers, antioxidants (e.g. ascorbic acid, etc.), buffers, preservatives, surfactants, chelating agents (e.g. EDTA, etc.) or binders, and the like. In addition, other low molecular weight polypeptides, proteins such as serum albumin, gelatin, and immunoglobulins, amino acids such as glycine, glutamine, asparagine, glutamic acid, aspartic acid, methionine, arginine and lysine, carbohydrates such as polysaccharides and monosaccharides or carbohydrates, sugar alcohols such as mannitol and sorbitol may be included. In the case of aqueous solutions for injection, for example, physiological saline, isotonic solutions containing glucose and other adjuvants, e.g. D-sorbitol, D-mannose, D-mannitol, sodium chloride, can be used in combination with suitable cosolvents, such as alcohols (ethanol and the like), polyols (propylene glycol, PEG and the like), non-ionic surfactants (polysorbate 80, polysorbate 20, poloxamer 188, HCO-50) and the like. In addition, by mixing hyaluronidase in the formulation, subcutaneous administration of larger amounts of fluid is also possible.

The binding molecules or antigen-binding fragments disclosed in the present disclosure can be used in combination with other drugs, and the active ingredients can be mixed together to form a single dosage unit, or they can be separately used as a dosage unit.

The term "effective amount" refers to the dosage of the drug formulation of the antibody or fragment disclosed in the present disclosure, which produces the expected effect in the treated patient after being administered in single or multiple dosages. The effective amount can be easily determined by attending physicians who are skilled in the field by considering various factors such as ethnic differences; weight, age, and health status; specific diseases involved; the severity of the disease; individual patient responses; specific antibodies administered; administration mode; the bioavailability characteristics of the administered formulation; selected medication regimen; the use of any accompanying therapy.

The term "kit" or "reagent kit" includes an effective amount of one or more of the pharmaceutical compositions of the present disclosure in unit dosage form. In some embodiments, the drug kit may comprise a sterile container; such containers may be boxes, ampoules, bottles, vials, tubes, bags, blister packs, or other suitable container forms known in the art. Such containers may be made of plastic, glass, laminated paper, metal foil, or other materials suitable for holding medicaments. In addition, the drug kit includes instructions for administering the pharmaceutical composition of the present disclosure to an individual. Methods for treating diseases using the pharmaceutical compositions of the present disclosure are generally included in the specification.

The term "individual" or "subject" used herein refers to any animal, such as a mammal or marsupial. Individuals of the present disclosure include, but are not limited to, humans, non-human primates (e.g. cynomolgus or rhesus monkeys or other types of macaques), mice, pigs, horses, donkeys, cattle, sheep, rats, and poultry of any species.

As used herein, the terms "disease", "condition" or "disorder" and the like refer to any alteration or disorder that impairs or interferes with the normal function of a cell, tissue, or organ. For example, such "diseases" include, but are not limited to a tumor, a pathogen infection, an autoimmune disease, a T-cell dysfunction disease, or a defect in immune tolerance (e.g. transplant rejection).

As used herein, the term "tumor" refers to a disease characterized by pathological proliferation of cells or tissues, and subsequent migration or invasion of other tissues or organs. Tumor growth is generally uncontrolled and progressive and does not induce or inhibit normal cell proliferation.

As used herein, the term "treatment" refers to clinical intervention in an attempt to alter an individual or to treat a cell-caused disease process, either prophylactically or clinically pathological. Therapeutic effects include but are not limited to, preventing the onset or recurrence of the disease, alleviating symptoms, diminishing any direct or indirect pathological consequences of the disease, preventing metastasis, slowing disease progression, amelioration or palliation of the disease state, relieving or improving prognosis, and the like.

### Examples

The present disclosure is further illustrated by the following specific examples. It should be understood that the examples are only used to illustrate the present disclosure and not to limit the scope of the present disclosure. Experimental procedures for which no specific conditions are indicated in the following examples are generally performed according to conventional conditions (such as those described in J. SAMBROOK et al. eds. Molecular Cloning: A Laboratory Manual, 3rd Ed. Science Press, 2002) or as recommended by the manufacturer.

### Example 1. Human DLL3 and Cynomolgus monkey DLL3 antigen information

The full-length amino acid sequence of human DLL3 (SEQ ID NO: 105) (Uniprot ID: Q9 NYJ7) used in the embodiment is shown below, which was purchased from Kactus Biosystems (Cat. No: DLL-HM103).

Note: the double underline part represents a signal peptide (1-26); the underline part represents an extracellular domain of DLL3 (27-492); the dotted line represents a transmembrane region (493-513); the italic part represents an intracellular region (514-618). The full-length amino acid sequence (SEQ ID NO: 106) (Uniprot ID: A0A2K5WSR4) of the Cynomolgus monkey DLL3 (cyno DLL3) used in the embodiment is shown below, which was purchased from Kactus Biosystems (Cat. No: CM103).

MVSPRMSRLLSQTVILALIFIPQARPAGVFELQIHSFGPGPGPGAPRSPCSARGPCRL
FFRVCLKPGLSEEAAESPCALGAALSARGPVYTEQPEAPAPDLPLPNGLLQVPFRD
AWPGTFSLIIETWREELGDQIGGPAWSLLARVTRRRRLAAGGPWARDIQRAGAWE
LRFSYRARCELPAVGTACTRLCRPRSAPSRCGPGLRPCAPLEDECEAPPVCRAGCS
LEHGFCEQPGECRCLEGWTGPLCMVPVSTSSCLGLRGPSSTTTGCLVPGPGPCDG
NPCANGGSCSETPGSFECTCPRGFYGLRCEVSGVTCADGPCFNGGLCVGGADPDS
AYICHCPPGFQGSNCEKRVDRCSLQPCRNGGLCLDLGHALRCRCRAGFAGPRCEH
DLDDCAGRACANGGTCVEGGGAHRCSCALGFGGRNCRERADPCAARPCAHGGR
CYAHFSGLVCACAPGYMGARCEFPVHPDGVSALPAAPPGLRPGDPQRYLLPPALG
LLVAAGVAGAALLLVHVRRRGHAQDAGSRLLAGTPEPSVHALPDALNNLRTQEGPG
DVPSSSVDWNRPEDVDSRGIYVISAPSIYAREVAMPLFPPLHTGRAGQRQNLLFPFPSSIL
SVK

Note: the double underline part represents a signal peptide (1-26); the underline part represents an extracellular domain of DLL3 (27-490); the dotted line represents a transmembrane region (491-513); the italic part represents an intracellular region (514-618).

### Example 2. Family member DLL1 and DLL4 antigen information

The full-length amino acid sequence (SEQ ID NO: 107) (Uniprot ID: O00548) of human DLL1 used in the embodiment is shown below, which was purchased from Sino Biological (Cat. No: 11635-H08H).

Note: the double underline part represents a signal peptide (1-17); the underline part represents an extracellular domain of DLL3 (18-545); the dotted line represents a transmembrane region (546-568); the italic part represents an intracellular region (569-723). The full-length amino acid sequence (SEQ ID NO: 108) (Uniprot ID: Q9NR61) of human DLL4 in the embodiment is shown below, which was purchased from Sino Biological (Cat. No: 10171-H08H).

Note: the double underline part represents a signal peptide (1-26); the underline part represents an extracellular domain of DLL3 (27-529); the dotted line represents a transmembrane region (530-550); the italic part represents an intracellular region (551-685).

### Example 3. Construction of heavy chain antibody immune library for camel immunity

Camels were immunized with the human DLL3 antigen (described in Example 1). The days of immunization were Day 0, Day 21, Day 35, and Day 49, respectively, for a total of 4 immunizations. Blood samples were collected on Day 28, 42, and 73, respectively, and the immune response was detected by ELISA. The titer of serum was detected to be greater than 1: 128000. After the completion of immunization, 100 mL of blood sample was collected again from immunized camels, and PBMC of camels were separated using the lymphocyte separation solution of Solarbio according to the manufacturer's instructions. After total RNA was extracted (OMEGA cell total RNA extraction kit), cDNA was synthesized using Takara PrimeScript^{™} II reverse transcription kit as a template, and a VHH gene fragment was amplified by nested PCR using designed specific primers. After recovery of the VHH fragment, the fragment was ligated into a pADL-23c phagemid vector by Sfi I digestion, and TG 1 electroporation competent cells were used to build immune library of DLL3 camels (capacity: 1.12E8).

### Example 4. Screening of camelid-derived anti-DLL3 positive clones

In order to obtain a positive antibody that can cross-bind to human DLL3 and Cynomolgus monkey DLL3, the above library was amplified and added to the M13K07 helper phage to assemble phage. 1 × 10¹² pfu camel immune library phage was added and incubated with biotinylated human DLL3 proteins (8 µg/mL) bound to magnetic beads for 1 h at room temperature. After washing with 0.05% PBST to remove unbound phage, the phage specifically bound to DLL3 was eluted with 100 mM triethylamine. After gradient dilution, log-phase growth of E. coli SS320 was infected and spread on an ampicillin plate overnight at 37°C. A single clone was picked for IPTG-induced expression and the supernatant was used for ELISA detection. The ELISA plate was coated with 2 µg/ml human DLL3 or Cynomolgus monkey DLL3 antigen respectively overnight at 4°C. The plate was washed with 0.05% PBST 3 times, then blocked with 5% skimmed milk for 1 h at room temperature, and washed with 0.05% PBST 3 times. Then 30 µL of induced supernatant was added to each well, and a culture medium was added to a negative control well. The plate was incubated for 1 h at room tempature. Finally, anti-Myc HRP detection was performed (VHH expressed by IPTG induction has his and c-Myc labels). The amino acid sequences of the five heavy chain antibody variable regions of the present disclosure were obtained by sequencing clones from the ELISA assay that bound human and Cynomolgus monkey DLL3 with OD450 values greater than 1.0 and ELISA OD450 ratios greater than 3 to the medium as negative control, with the results shown in Table 1:

**Table 1 Amino acid sequences of five camelid-derived anti-DLL3 heavy chain antibody variable region**

| Clone No. | SEQ ID NO | Variable Heavy Chain (VHH) |
|---|---|---|
| DLL3-3 | 1 | DVQLVESGGGSVQAGGSLKLSCKSPTYTISSGYMGWF RQAPGKEREGVAAIYIGGSTTLYADSVKGRFTISADNAE KTVYLQMNTLKPEDSAMYYCAAQLRPNSAYHPLDGR KYNYWGQGTQVTVSS |
| DLL3-12 | 2 | QVQLVESGGGLVQPGESLRLSCAGSGFAFSSYDMHWV RQAPGKDFEWVSSISRDGRGPRYADFVKGRFTISKDNG RNMLYLQLNSLEIEDTAMYYCSKGYPIMGGTTQGTQV TVSS |
| DLL3-26 | 3 | QVQLVESGGGSVQAGGSLRLSCAASGDIYSSSYVGWF RQAPGKEREGVAIIYTSGDSTYYANSVKGRFTISQDKA KKTLYLQMNSLKPEDTAMYYCAARFAIDNSNYWGQG TQVTVSS |
| DLL3-122 | 4 | QVQLVESGGGSVQAGGSLRLSCTASGDTYRSYCMGWF RKAPGKEREGVADIVSDGSTSYADSVKGRFTISKDNAK NTLYLQMNSLKPEDTAMYYCAVDRGGSGGYCYTGRY DYWGQGTQVTVSS |
| DLL3-276 | 5 | QVQLVESGGGSVQAGGSLNLSCATSGSTASTTYMGWF RQAPGKGREGVAIIYTARDNPWYANSVKGRFIISQDNA KKTLYLQMNTLKPEDTATYYCAATLANPTRTAWGQGT LVTVSS |

On the basis of the above amino acid sequences, the CDR and FR of antibody variable regions were divided using the Kabat numbering rule, and the composition of 3 CDR sequences of each antibody is shown in Table 2 below.

**Table 2 CDR sequences of five camelid-derived anti-DLL3 heavy chain antibodies**

| Clone No. | | HCDR1 | HCDR2 | HCDR3 |
|---|---|---|---|---|
| DLL3-3 | SEQ ID NO | 6 | 7 | 8 |
| | Amino Acid Sequence | SGYMG | AAIYIGGSTTLYADSV KG | QLRPNSAYHPLDGRKY NY |
| DLL3-12 | SEQ ID NO | 9 | 10 | 11 |
| | Amino Acid | SYDMH | SSISRDGRGPRYADFV KG | GYPIMGG |
| | Sequence | | | |
| DLL3-26 | SEQ ID NO | 12 | 13 | 14 |
| | Amino Acid Sequence | SSYVG | AIIYTSGDSTYYANSV KG | RFAIDNSNY |
| DLL3-122 | SEQ ID NO | 15 | 16 | 17 |
| | Amino Acid Sequence | RSYCM G | ADIVSDGSTSYADSV KG | DRGGSGGYCYTGRYD Y |
| DLL3-276 | SEQ ID NO | 18 | 19 | 20 |
| | Amino Acid Sequence | TTYMG | IIYTARDNPWYANSV KG | TLANPTRTA |

### Example 5. Construction of camelid-derived anti-DLL3 chimeric antibodies and their transient transfection expression in eukaryotic cells

The gene fragment of interest generated after splicing the sequenced heavy chain antibody variable region of the present disclosure with the human IgG1 constant region was cloned into a pTT5 expression vector to prepare a transfection-grade expression plasmid. The heavy chain antibody variable region can be linked to the human IgG1 constant region by a linking short peptide, i.e. to form: heavy chain antibody variable region-linking short peptide-human IgG1 constant region. The linking short peptide sequence used in this example was GGGGS.

The introduced human IgG1 constant region sequence (SEQ ID NO: 109) is as follows:

DKTHTCPPCPAPELLGGPSVFLFPPKPKDTLMISRTPEVTCVVVDVSHEDPEVKFN

WYVDGVEVHNAKTKPREEQYNSTYRVVSVLTVLHQDWLNGKEYKCKVSNKALP
APIEKTISKAKGQPREPQVYTLPPSRDELTKNQVSLTCLVKGFYPSDIAVEWESNGQ
PENNYKTTPPVLDSDGSFFLYSKLTVDKSRWQQGNVFSCSVMHEALHNHYTQKSL
SLSPG

Expi293F^{™} cells (Thermo Fisher Scientific) were cultured in a serum-free medium, seeded in a shake flask (Corning Inc.), and cultured on a 37°C, 8% CO₂ shaker. After adjusting the cell density, the recombinant expression vector containing the gene fragment of interest and PEI transfection reagent were mixed in an appropriate ratio and added into a cell culture flask. After 6 days of cell culture, the expression supernatant was collected, centrifuged at high speed to remove cell debris, and subjected to affinity purification using a Protein A column. The column was rinsed with PBS until the A280 reading dropped to baseline. The protein of interest was eluted with an acidic eluent at pH 3.0-pH 3.5 and neutralized with 1M Tris-HCl, pH 8.0-9.0. After the eluted sample was appropriately concentrated, the solution was changed to PBS and aliquoted for later use. Final purified chimeric antibodies were subjected to SDS-PAGE and HPLC purity analysis and A280 concentration determination.

### Example 6. Binding of camelid-derived anti-DLL3 chimeric antibodies to DLL3-expressing cells

HEK293 cells (from the Chinese Academy of Sciences) were transfected with the full-length gene of cyno-DLL3 antigen (see Example 1 for the sequence), for cell culture and passage. Monoclonal selection was performed, and the expression level of DLL3 protein in the monoclonal cells were identified by flow cytometry. The culture was expanded and stored in storage for later use.

SHP-77 and HEK293-cyno DLL3 cells were cultured. The culture medium for SHP-77 cells were RPMI1640+10% FBS, and the culture medium for HEK293 cyno DLL3 cells were DMEM+10% FBS+200 µg/ml Hygromycin. The cells were cultured in a T75 cell culture flask at a 37°C in a 5% CO₂ incubator. When using cells, they are washed with sterile DPBS, digested with 0.25% trypsin EDTA for about 5 minutes, and then stopped with a complete medium.

The digested cells were centrifuged at 1000 rpm for 5 min at room temperature. The supernatant was discarded and the cells were resuspended in 100 µL of 1% BSA (in PBS). The cells were counted and adjusted to a cell density of 1E6/m. The dilute cells were seeded in a 96-well round-bottom culture plate (corning 3799), and centrifuged at 1500 rpm for 5 min at 4°C. The supernatant was discarded and the cells were stored at 4°C until use. Antibody samples to be tested were diluted with 1% BSA (in PBS) at a starting concentration of 100 nM, downwards by a 10-fold gradient for 7 concentrations. The cells were resuspended with the diluted antibody at 100 µL/well and incubated for 1 hour at 4°C. The cells were centrifuged at 1500 rpm for 5 minutes at 4°C and the supernatant was discarded. The cells were resuspended and washed with 160 µL of 1%BSA (in PBS), centrifuged at 1500 rpm for 5 minutes at 4°C. The supernatant was discarded. The secondary antibody (goat anti human IgG Fc PE) was diluted 1: 200 with 1% BSA (in PBS) according to the manufacturer's instructions and the cells were resuspended with the diluted secondary antibody, 100 µL/well, and incubated for 0.5 h at 4°C. The cells were centrifuged at 1500 rpm for 5 minutes at 4°C and the supernatant was discarded. The cells were resuspended and washed with 160 µL of 1%BSA (in PBS), centrifuged at 1500 rpm for 5 minutes at 4°C. The supernatant was discarded. The cells were resuspended in 100 µL of 1% BSA (in PBS), and filtered across a 300 mesh gauze. The mean fluorescence intensity of the PE channels was measured by flow cytometry.

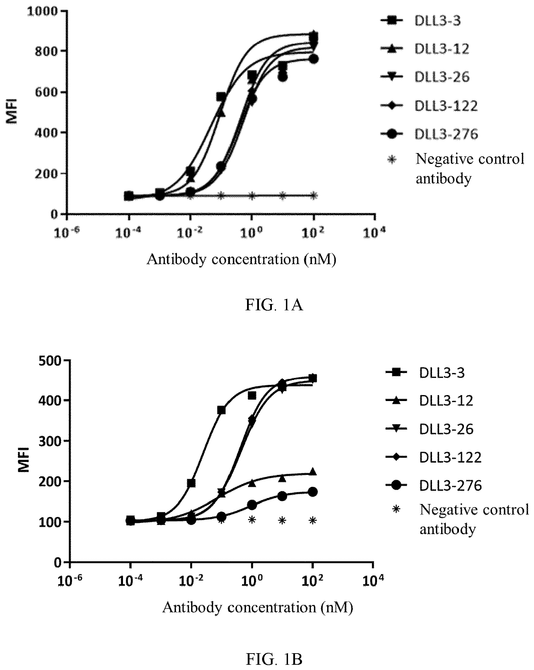

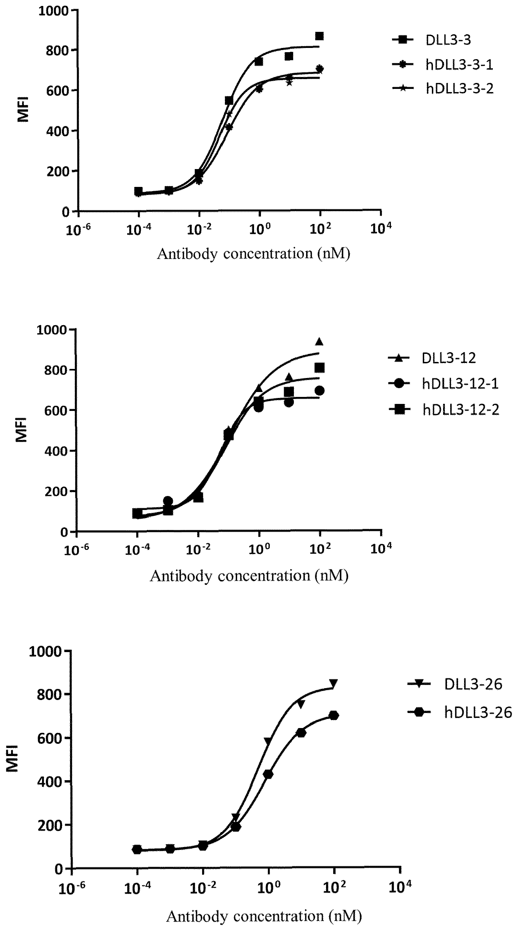

The FCS file was exported from the flow cytometer. The mean fluorescence intensity of the PE channel (hereinafter referred to as MFI) of each sample was analyzed with the Flowjo software. The analyzed mean fluorescence intensity was imported into Graphpad to analyze the median-binding concentration (hereinafter referred to as EC50) of antibody and cell and the top mean fluorescence intensity (Top MFI). The results are shown in Table 3 and FIG. 1.

**Table 3 Binding of anti-DLL3 chimeric antibodies to DLL3-expressing cells**

| Clone No. | SHP-77 | | HEK293 - cyno DLL3 | |
|---|---|---|---|---|
| | EC₅₀ (nM) | Top Mean Fluorescence Intensity (Top MFI)) | EC₅₀ (nM) | Top Mean Fluorescence Intensity (Top MFI)) |
| Negative control antibody | Unfitted | 92 | Unfitted | 104 |
| DLL3-3 | 0.047 | 874 | 0.025 | 438 |
| DLL3-12 | 0.092 | 886 | 0.073 | 220 |
| DLL3-26 | 0.519 | 821 | 0.439 | 450 |
| DLL3-122 | 0.427 | 846 | 0.415 | 460 |
| DLL3-276 | 0.374 | 763 | 0.897 | 175 |

### Example 7. Humanization Design of Camel Derived Anti-DLL3 Heavy Chain Antibodies

The germline gene sequences with high homology to the candidate heavy chain antibody were selected as the VHH transplantation framework template through sequence alignment. After grafting the CDR region of the candidate antibody to the framework of the variable region of the selected human antibody, reverse mutation of individual amino acids was performed to obtain a humanized antibody. The amino acid sequence of the humanized variable region is shown in Table 4.

**Table 4 Amino acid sequence of variable region of 7 camelid-derived humanized anti-DLL3 antibodies**

| Clone No. | SEQ ID NO | Variable Heavy Chain (VHH) |
|---|---|---|
| hDLL3-3 -1 | 21 | EVQLVESGGGLVQPGGSLRLSCAASTYTISSGYMGWFRQAPG KEREGVAAIYIGGSTTLYADSVKGRFTISRDNSKNTLYLQMNS |
| | | LRAEDTAVYYCAAQLRPNSAYHPLDGRKYNYWGQGTLVTVS S |
| hDLL3-3 -2 | 22 | EVQLVESGGGLVQPGGSLRLSCKSPTYTISSGYMGWFRQAPG KEREGVAAIYIGGSTTLYADSVKGRFTISRDNSKNTLYLQMNS LRAEDTAVYYCAAQLRPNSAYHPLDGRKYNYWGQGTLVTVS S |
| hDLL3-12 -1 | 23 | QVQLVESGGGVVQPGRSLRLSCAASGFAFSSYDMHWVRQAP GKGLEWVSSISRDGRGPRYADSVKGRFTISRDNSKNTLYLQM NSLRAEDTAVYYCSKGYPIMGGTTQGTLVTVSS |
| hDLL3-12 -2 | 24 | QVQLVESGGGVVQPGRSLRLSCAASGFAFSSYDMHWVRQAP GKDFEWVSSISRDGRGPRYADSVKGRFTISRDNSKNTLYLQM NSLRAEDTAVYYCSKGYPIMGGTTQGTLVTVSS |
| hDLL3-26 | 25 | EVQLVESGGGLVQPGGSLRLSCAASGDIYSSSYVGWFRQAPG KEREGVAIIYTSGDSTYYADSVKGRFTISRDNSKNTLYLQMNS LRAEDTAVYYCAARFAIDNSNYWGQGTLVTVSS |
| hDLL3-122 | 26 | EVQLVESGGGLVQPGGSLRLSCAASGDTYRSYCMGWFRQAP GKEREGVADIVSDGSTSYADSVKGRFTISRDNSKNTLYLQMNS LRAEDTAVYYCAVDRGGSGGYCYTGRYDYWGQGTLVTVSS |
| hDLL3-276 | 27 | QVQLVESGGGVVQPGGSLRLSCAASGSTASTTYMGWFRQAP GKGREGVAIIYTARDNPWYADSVKGRFTISRDNSKNTLYLQM NSLRAEDTAVYYCAATLANPTRTAWGQGTLVTVSS |

### Example 8. Preparation of camelid-derived humanized anti-DLL3 antibodies

As described in Example 5, the gene fragment of interest generated after splicing the variable region of the humanized antibody with the constant region of human IgG1 was cloned into the pTT5 expression vector to prepare a transfection-grade expression plasmid. Expi293F^{™} cells (Thermo Fisher Scientific) were cultured in a serum-free medium, seeded in a shake flask (Corning Inc.), and cultured on a 37°C, 8% CO₂ shaker. After adjusting the cell density, the recombinant expression vector containing the gene fragment of interest and PEI transfection reagent were mixed in an appropriate ratio and added into a cell culture flask. After 6 days of cell culture, the expression supernatant was collected, centrifuged at high speed to remove cell debris, and subjected to affinity purification using a Protein A column. The column was rinsed with PBS until the A280 reading dropped to baseline. The protein of interest was eluted with an acidic eluent at pH 3.0-pH 3.5 and neutralized with 1M Tris-HCl, pH 8.0-9.0. After the eluted sample was appropriately concentrated, the solution was changed to PBS and aliquoted for later use. The final purified humanized antibody was subjected to SDS-PAGE and HPLC purity analysis and A280 concentration determination.

### Example 9. In vitro cell binding validation of camelid-derived humanized anti-DLL3 antibodies

SHP-77 and HEK293-cyno DLL3 cells were cultured. The culture medium for SHP-77 cells were RPMI1640+10% FBS, and the culture medium for HEK293 cyno DLL3 cells were DMEM+10% FBS+200 µg/ml Hygromycin. The cells were cultured in a T75 cell culture flask at a 37°C 5% CO₂ incubator. When using cells, they are washed with sterile DPBS, digested with 0.25% trypsin EDTA for about 5 minutes, and then stopped with a complete medium.

The digested cells were centrifuged at 1000 rpm for 5 min at room temperature. The supernatant was discarded and the cells were resuspended in 100 µL of 1% BSA (in PBS). The cells were counted and adjusted to a cell density of 1E6/m. The dilute cells were seeded in a 96-well round-bottom culture plate (corning 3799), and centrifuged at 1500 rpm for 5 min at 4°C. The supernatant was discarded and the cells were stored at 4°C until use. Antibody samples to be tested were diluted with 1% BSA (in PBS) at a starting concentration of 100 nM, downwards by a 10-fold gradient for 7 concentrations. The cells were resuspended with the diluted antibody at 100 µL/well and incubated for 1 hour at 4°C. The cells were centrifuged at 1500 rpm for 5 minutes at 4°C and the supernatant was discarded. The cells were resuspended and washed with 160 µL of 1%BSA (in PBS), centrifuged at 1500 rpm for 5 minutes at 4°C. The supernatant was discarded. The secondary antibody (goat anti human IgG Fc PE) was diluted 1: 200 with 1% BSA (in PBS) according to the manufacturer's instructions and the cells were resuspended with the diluted secondary antibody, 100 µL/well, and incubated for 0.5 h at 4°C. The cells were centrifuged at 1500 rpm for 5 minutes at 4°C and the supernatant was discarded. The cells were resuspended and washed with 160 µL of 1%BSA (in PBS), centrifuged at 1500 rpm for 5 minutes at 4°C. The supernatant was discarded. The cells were resuspended in 100 µL of 1% BSA (in PBS), and filtered across a 300 mesh gauze. The mean fluorescence intensity of the PE channels was measured by flow cytometry.

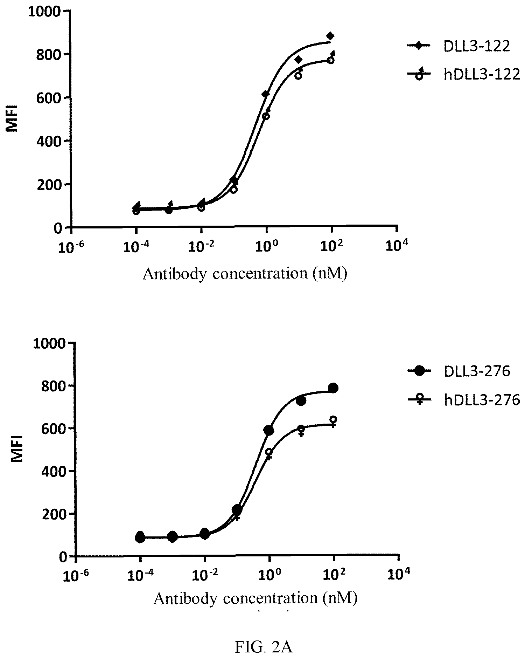

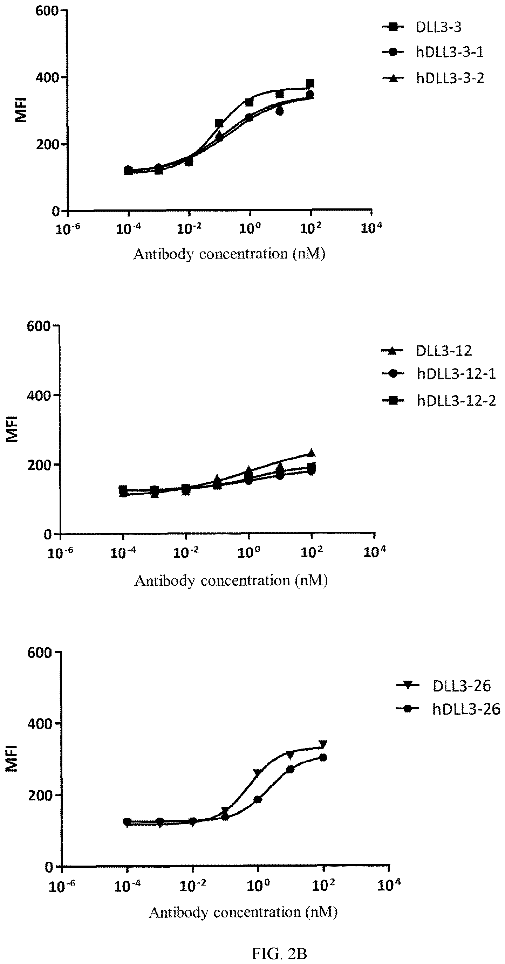

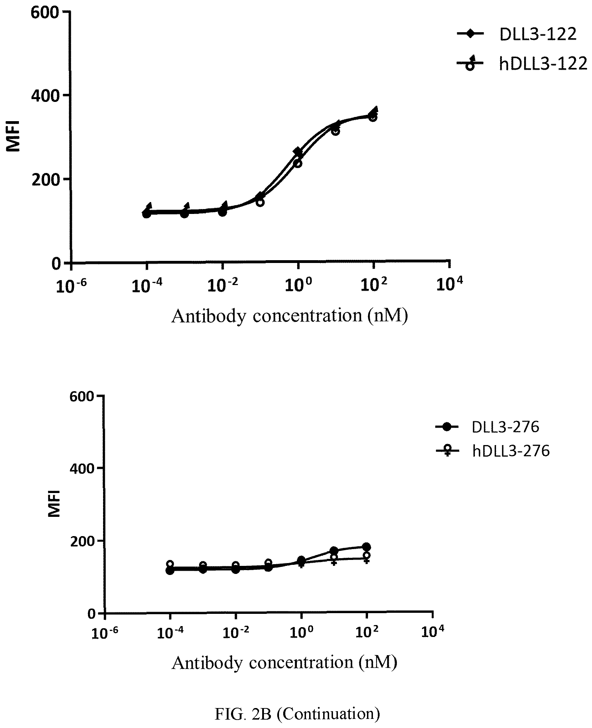

The FCS file was exported from the flow cytometer. The mean fluorescence intensity of the PE channel (hereinafter referred to as MFI) of each sample was analyzed with the Flowjo software. The analyzed mean fluorescence intensity was imported into Graphpad to analyze the median-binding concentration (hereinafter referred to as EC50) of antibody and cell and the top mean fluorescence intensity (Top MFI). The results are shown in Table 5 and Figure 2.

**Table 5 Binding of humanized anti-DLL3 antibodies to DLL3-expressing cells**

| Clone No. | SHP-77 | | HEK293 - cyno DLL3 | |
|---|---|---|---|---|
| | EC₅₀ (nM) | Top Mean Fluorescence Intensity (Top MFI)) | EC₅₀ (nM) | Top Mean Fluorescence Intensity (Top MFI)) |
| DLL3-3 | 0.061 | 864 | 0.08 | 379 |
| hDLL3-3 -1 | 0.086 | 702 | 0.22 | 346 |
| hDLL3-3 -2 | 0.049 | 691 | 0.15 | 344 |
| DLL3-12 | 0.114 | 935 | 1.38 | 232 |
| hDLL3-12 -1 | 0.052 | 690 | 2.36 | 178 |
| hDLL3-12 -2 | 0.081 | 804 | 1.35 | 190 |
| DLL3-26 | 0.499 | 841 | 0.54 | 336 |
| hDLL3-26 | 0.786 | 696 | 2.24 | 301 |
| DLL3-122 | 0.478 | 877 | 0.58 | 351 |
| hDLL3-122 | 0.589 | 778 | 0.99 | 351 |
| DLL3-276 | 0.396 | 782 | 2.08 | 179 |
| hDLL3-276 | 0.389 | 622 | 1.01 | 148 |

### Example 10. Biacore Affinity Experiment of a camelid-derived humanized anti-DLL3 antibody

The affinity and kinetic properties of the humanized anti-DLL3 antibody to human DLL3 were analyzed using a Biacore 8K instrument. CM5 chips were activated with EDC and NHS, followed by immobilization of anti-human Fc murine mAb and blocked with ethanolamine.

To determine the affinity to human DLL3 and kinetic properties, the DLL3 humanized antibody was diluted to 0.2 µg/mL with HBS-EP + (10 mM HEPES, pH 7.4, 150 mM NaCl, 3 mM EDTA, 0.05% P20) buffer and captured at a flow rate of 10 µL/min for 45 s. Human DLL3 was diluted two-fold serially to serial concentrations (100 nM-0.39 nM) and associated for 90 s and dissociated for 600 s at a flow rate of 50 µL/min.

After completion of each cycle of the experiment, the captured antibody was removed together with the antigen by washing with a 3M MgCl₂ solution at a flow rate of 30 µL/min for 30 s to complete the regeneration of the chip. The raw data were analyzed using Biacore Insight Evaluation Software (3.0.12.15655) software and fitted with a (1: 1) Langmuir model. The results were shown in Table 6.

**Table 6. Affinity test results of humanized anti-DLL3 antibodies with human DLL3 antigen proteins**

| Clone No. | ka (1/Ms) | kd(1/s) | KD(M) |
|---|---|---|---|
| DLL3-3 | 2.15E+06 | 2.74E-04 | 1.27E-10 |
| hDLL3-3 -1 | 1.91E+06 | 5.80E-04 | 3.04E-10 |
| hDLL3-3 -2 | 1.95E+06 | 7.76E-04 | 3.99E-10 |
| DLL3-26 | 3.82E+05 | 1.23E-05 | 3.22E-11 |
| hDLL3-26 | 2.10E+05 | 3.55E-04 | 1.69E-09 |
| DLL3-122 | 9.32E+04 | 3.92E-04 | 4.21E-09 |
| hDLL3-122 | 9.85E+04 | 4.66E-04 | 4.73E-09 |

### Example 11. Binding experiments of camelid-derived humanized anti-DLL3 antibodies to the family proteins DLL1 and DLL4

The antigenic proteins human DLL1 (SinoBiological, cat. No. 11635-H08H) or human DLL4 (SinoBiological, cat. No. 10171-H08H) were dissolved in 1 × PBS at a concentration of 1 µg/mL. The antigen was then added to a high-affinity ELISA plate (Biolegend, cat. No. 423501) at 100 µL/well and left overnight at 4°C. The antigen was washed three times with PBST, 300 µL/well. The antigen was blocked with 1% BSA (in PBST), 200 µL/well, and incubated at 37°C for 1.5 hours.

Antibodies to be tested were diluted with 1% BSA (in PBS) at a starting concentration of 100 nM, downwards by a 10-fold gradient for 7 concentrations. The plates were washed three times with PBST, 300 µL/well. The diluted antibody was added to an ELISA plate at 100 µL/well and incubated at room temperature for 2 hours. The antibodies were washed three times with PBST, 300 µL/well. The secondary antibodies (goat anti-human IgG Fc for DLL4 (HRP), 1: 20000 dilution; HRP goat anti-mouse IgG (H + L) for DLL1, 1: 10000 dilution) were diluted with 1% BSA in PBST. The diluted secondary antibodies were added to the ELISA plate at 100 µL/well and incubated at room temperature for 1 hour. The plates were washed 6 times with PBST, 300 µL/well. The developing solution (TMA and TMB 1:1 mixed) was prepared. The developing solution was added to the plate, 100 µL/well, and incubated in the dark for 5 min. 50 µL of ELISA stop solution was added to the plate and shaken well.

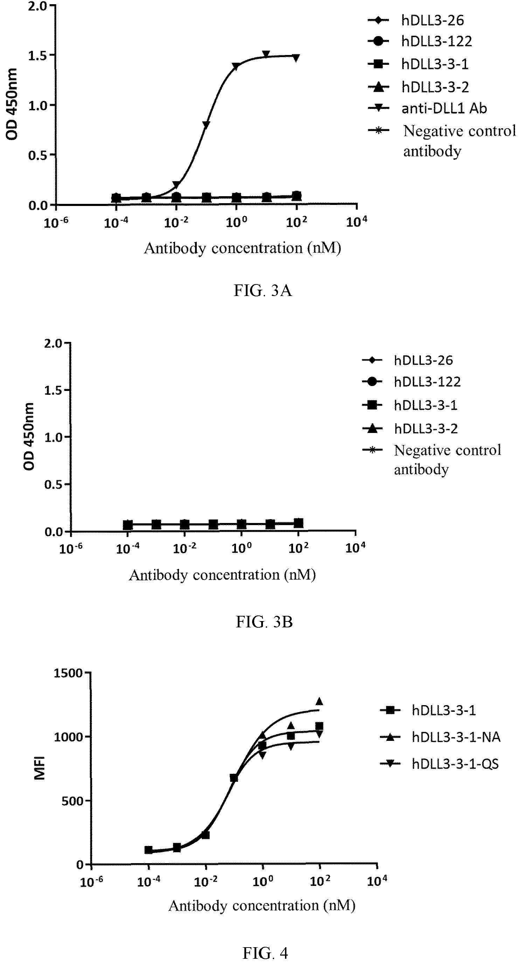

OD450s were read on the envision and plotted on the GraphPad for EC₅₀ values. The results are shown in FIG. 3, which show that the antibodies tested did not non-specifically bind to either of the family proteins DLL1 and DLL4.

### Example 12. Preparation of variants of Camelid-derived humanized anti-DLL3 antibodies

After post-translational modification (PTM) analysis of the heavy chain antibody of the present disclosure, it was found that there was one deamidation site in the variable region of hDLL3-3-1; and site-directed mutation for a single site was performed on amino acid 103 to prepare two mutants of hDLL3-3-1: hDLL3-3-1-NA and hDLL3-3-1-QS, respectively. The variable region amino acid sequences of the two variants are shown in Table 7.

**Table 7 Amino acid sequences of hDLL3-3-1 humanized antibody variants**

| Clone No. | SEQ ID NO | Variable Heavy Chain (VHH) | SEQ ID NO | Heavy Chain CDR3 |
|---|---|---|---|---|
| hDLL3-3-1-NA | 28 | EVQLVESGGGLVQPGGSLR LSCAASTYTISSGYMGWFR QAPGKEREGVAAIYIGGST TLYADSVKGRFTISRDNSK NTLYLQMNSLRAEDTAVY YCAAQLRPNAAYHPLDGR KYNYWGQGTLVTVSS | 110 | QLRPNAAY HPLDGRK YNY |
| hDLL3-3-1-QS | 29 | EVQLVESGGGLVQPGGSLR LSCAASTYTISSGYMGWFR QAPGKEREGVAAIYIGGST TLYADSVKGRFTISRDNSK NTLYLQMNSLRAEDTAVY YCAAQLRPQSAYHPLDGR KYNYWGQGTLVTVSS | 111 | QLRPQSAY HPLDGRK YNY |

The two variants were prepared as described in Example 5. After transient expression in mammalian cell lines, affinity assays were performed using SHP-77 cells. The results shown in Table 8 and FIG. 4 show that the two variants were able to eliminate the risk of post-translational modification without significant changes in binding at the cellular level. See Table 9 for affinity assay results using human DLL3 antigen proteins.

**Table 8 Binding of hDLL3-3-1 humanized antibody variants to DLL3-expressing cells (SHP-77)**

| Clone No. | SHP-77 | |
|---|---|---|
| | EC₅₀ (nM) | Top Mean Fluorescence Intensity (Top MFI)) |
| hDLL3-3 -1 | 0.068 | 1076 |
| hDLL3-3-1-NA | 0.104 | 1271 |
| hDLL3-3-1-QS | 0.055 | 1009 |

**Table 9 Affinity test results of hDLL3-3-1 humanized antibody variants with human DLL3 antigen protein**

| Clone No. | ka (1/Ms) | kd(1/s) | KD(M) |
|---|---|---|---|
| hDLL3-3 - 1 | 1.36E+06 | 6.14E-04 | 4.50E-10 |
| hDLL3-3-1-NA | 1.29E+06 | 1.35E-04 | 1.05E-10 |
| hDLL3-3-1-QS | 1.22E+06 | 6.11E-03 | 5.02E-09 |

### Example 13. Obtaining mouse hybridoma-derived anti-DLL3 antibodies

Anti-DLL3 monoclonal antibodies were generated by immunizing mice. The experiment used Swiss Webster white mice, female, 6 weeks old (Charles River). Housing: SPF grade. After the mice were purchased, they were kept in a laboratory environment for 1 week. The light/dark cycle was 12/12 hours, the temperature was 20-25°C; humidity was 40-60%. The immunizing antigen was His-tagged human DLL3 recombinant protein (huDLL3-His). Titermax (sigma Lot Num: T2684) was an adjuvant. The antigen to adjuvant (titermax) ratio was 1:1, the antigen was emulsified and inoculated on days 0, 14, 35, and 56, and boosted 3 days before splenocyte fusion. During this period, the ELISA and FACS methods were used to detect mouse serum and determine the antibody titer in the mouse serum. After the fifth immunization, mice with high and plateau antibody titer in serum were selected for splenocyte fusion. Splenic lymphocytes were fused with myeloma cells Sp2/0 cells (ATCC^{®} CRL-8287^{™}) using an optimized electrofusion procedure to obtain hybridoma cells.

After the fused hybridoma cells are cultured for 7-14 days, the culture medium supernatant was taken. The hybridoma supernatant was subjected to antibody screening using DLL3 recombinant proteins and an ELISA experiment. The obtained positive antibody strain was further screened with stably transfected CHO-K1 cells expressing DLL3 to exclude nonspecific antibody-binding hybridoma strains by comparing with blank CHO-K1 cells and subjected to screening using flow sorting method, thereby selecting hybridomas that bind to the recombinant protein and also bind to the antigen expressed by the cells. Hybridoma cells were harvested in the log phase and RNA was extracted with Trizol (Invitrogen, 15596-018) and reverse transcribed (PrimeScript^{™} Reverse Transcriptase, Takara # 2680A). The reverse transcribed cDNA was subjected to PCR amplification using a mouse Ig-Primer Set (Novagen, TB326 Rev. B 0503) and sequenced to obtain the amino acid sequences of the variable regions of the 10 monoclonal antibodies of the present disclosure, as shown in Table 10A.

**Table 10A Amino acid sequences of 10 mouse hybridomas-derived anti-dll3 monoclonal antibody variable regions**

| Clone No. | | Variable Heavy Chain (VH) | Variable Light Chain (VL) |
|---|---|---|---|
| 26C4D2 | SEQ ID NO | 30 | 31 |
| | Amino Acid | QVQLQQPGAELVKPGASVKL SCKASGYSFTSYWMHWVKQ | DIQMTQTTSSLSASLGDRVTI SCSASQGISNYLNWYQQKP |
| | Sequence | RPGQGLEWIGMIHPTLGDTNY NEKFKSKATLTVDKSSSTAYM ELSSLTSEDSAVYYCARLGSLS MMDYWGQGTSVTVSS | DGTVKLLIYYTSSLHSGVPA RFSGSGSGTDYSLTISNLEPE DIAIYYCQQYSKFPYTFGGG TKLEIK |
| 39E2D11-1 | SEQ ID NO | 32 | 33 |
| | Amino Acid Sequence | QVQLQQSGAELVKPGASVKM SCKASGYTFISYWITWVKQRP GQGLEWIGDIYPGSGSTTNYN EKFKSKATLTVDTSSSTAYMQ LSSLTSEDSAVYYCARETTVG GAYAMDYWGQGTSVTVSS | DIVLTQSPATLSVTPGDSVSL SCRASQSINNNLHWYQQKS HESPRLLIKYVSQSISGIPSRF SGSGSGTDFTLTINSVETEDF GMYFCQQTNSWPLTFGAGT KLELK |
| 39E2D11-2 | SEQ ID NO | 34 | 33 |
| | Amino Acid Sequence | QVQLKQSGPGLVQPSQSLSIT CTVSGFSLTSYGVHWVRQSPG KGLEWLGVIWSGGSTDYNAA FISRLSISKDNPKSQVFFKMNS LQADDTAIYYCARENYYGNS LWFFDVWGTGTTVTVSS | DIVLTQSPATLSVTPGDSVSL SCRASQSINNNLHWYQQKS HESPRLLIKYVSQSISGIPSRF SGSGSGTDFTLTINSVETEDF GMYFCQQTNSWPLTFGAGT KLELK |
| 40G3D1 | SEQ ID NO | 35 | 36 |
| | Amino Acid Sequence | QVQLQQSGAELVKPGASVKIS CKASGYAFSSQWMNWVKQR PGKGLEWIGQIYPGNGDTNY NGKFKGKATLTADKSSSTAYI QLSSLTSEDSAVYFCARWFAY WGQGTLVTVSA | DIVLTQSPVTLSVTPGDSVSL SCRASQSVRNNLHWYQQKS HESPRLLIKYVSQSISGIPSRF SGSGSGTDFTLSINSVETEDF GVYFCQQSNSWPLTFGAGT KLELK |
| 46A5A4 | SEQ ID NO | 37 | 38 |
| | Amino Acid Sequence | DIQMTQTTSSLSASLGDRVT FTCSASQGISNYLNWYQQK PDGTIKLLIYYTSSLHSGVPS RFSGSGSGTDYSLTISNLEPE DIATYYCQQYSKLPYTFGGG TKLEIK | DIQMTQTTSSLSASLGDRVT FTCSASQGISNYLNWYQQK PDGTIKLLIYYTSSLHSGVPS RFSGSGSGTDYSLTISNLEPE DIATYYCQQYSKLPYTFGGG TKLEIK |
| 47E12D12-1 | SEQ ID NO | 39 | 40 |
| | Amino Acid Sequence | EVQLVESGGGLVKPGGSLKLS CAASGFTFSDYGMHWVRQAP EKGLEWVAYISSGSSTIYYADT VKGRFTISRDNAKNTLFLQMT SLRSEDTAMYYCARNSRGFA YWGQGTLVTVSA | DIVLTQSPAIMSASPGEKVT MTCSASSSVSYMYWYQQKP GSSPRLLIYDTSNLASGVPV RFSGSGSGTSYSLTISRMEAE DAATYYCQQWSSYPRTFGG GTKLEIK |
| 47E12D12-2 | SEQ ID NO | 39 | 41 |
| | Amino Acid Sequence | EVQLVESGGGLVKPGGSLKLS CAASGFTFSDYGMHWVRQAP EKGLEWVAYISSGSSTIYYADT VKGRFTISRDNAKNTLFLQMT SLRSEDTAMYYCARNSRGFA YWGQGTLVTVSA | DIVLIQSPTIMSASPGEKVTM TCSASSSVSSMHWYQQKSG TSPKRWIYDTSKLASGVPAR FSGSGSGTSYSLTISTMEAED AATYYCQQWNSYHLTFGAG TKLELK |
| 48B6D7 | SEQ ID NO | 42 | 43 |
| | Amino Acid | QVQLQQPGAELVKPGTSVKLS CEASGYTFSNYWMQWVRQR | DIQMTQTTSSLSVSLGDRVTI NCSASQGISNYLNWYQQKP |
| | Sequence | PGQGLEWIGMILPNSDITNYN ENFQTKATLTVDKSSSTAYMQ LSSLTSEDSAVYYCARQARYS AMDYWGQGTSVTVSS | DGTVKLLIYYTSNLHSGVPS RFSGSGSGTDYSLTISNLEPE DIATYYCQHYSKFPYTFGGG TKLEIK |
| 45E4D7 | SEQ ID NO | 44 | 36 |
| | Amino Acid Sequence | QVQLQQPGAELVKPGTSVKLS CKASGYTFTSHWITWVKQRP GQGLEWIGDIYPISGSTNNNE KFRNKATLTVDTSSSTAYMQL SSLTSEDSAVYFCAKIITVGGA YVMDYWGQGTSVTVSS | DIVLTQSPVTLSVTPGDSVSL SCRASQSVRNNLHWYQQKS HESPRLLIKYVSQSISGIPSRF SGSGSGTDFTLSINSVETEDF GVYFCQQSNSWPLTFGAGT KLELK |
| 27E10C5 | SEQ ID NO | 35 | 45 |
| | Amino Acid Sequence | QVQLQQSGAELVKPGASVKIS CKASGYAFSSQWMNWVKQR PGKGLEWIGQIYPGNGDTNY NGKFKGKATLTADKSSSTAYI QLSSLTSEDSAVYFCARWFAY WGQGTLVTVSA | DIVMTQSPSSLAMSVGQKV TMNCKSSQSLLNSSHQKNY LAWYQQKPGQSPKLLVYFA STRESGVPDRFIGSGSGTDFT LTISSVQAEDLADYFCQQHY STPWTFGGGTKLEIK |

On the basis of the above amino acid sequences, the CDR and FR of antibody variable regions were divided using the Kabat numbering rule, and the composition of 6 CDR sequences of each antibody is shown in Table 10B below.

**Table 10B CDR Sequences of 10 mouse hybridomas-derived anti-DLL3 monoclonal antibodies**

| Clone No. | | CDR1 | | CDR2 | | CDR3 | |
|---|---|---|---|---|---|---|---|
| | | SEQ ID NO | Amino Acid Sequence | SEQ ID NO | Amino Acid Sequence | SEQ ID NO | Amino Acid Sequence |
| 26C4D2 | Heavy Chain | 57 | SYWMH | 58 | MIHPTLGDTNYNE KFKS | 59 | LGSLSMMDY |
| | Light chain | 60 | SASQGISNY LN | 61 | YTSSLHS | 62 | QQYSKFPYT |
| 39E2D11-1 | Heavy Chain | 63 | SYWIT | 64 | DIYPGSGSTTNYNE KFKS | 65 | ETTVGGAYA MDY |
| | Light Chain | 66 | RASQSINN NLH | 67 | YVSQSIS | 68 | QQTNSWPLT |
| 39E2D11-2 | Heavy Chain | 69 | SYGVH | 70 | VIWSGGSTDYNAA FIS | 71 | ENYYGNSLW FFDV |
| | Light Chain | 66 | RASQSINN NLH | 67 | YVSQSIS | 68 | QQTNSWPLT |
| 40G3D1 | Heavy Chain | 72 | SQWMN | 73 | QIYPGNGDTNYNG KFKG | 74 | WFAY |
| | Light Chain | 75 | RASQSVRN NLH | 76 | YVSQSIS | 77 | QQSNSWPLT |
| 46A5A4 | Heavy Chain | 78 | DHTIH | 79 | YIYPRDGYTMYNE KFKG | 80 | AFHALDY |
| | Light chain | 81 | SASQGISNY LN | 82 | YTSSLHS | 83 | QQYSKLPYT |
| 47E12D12 -1 | Heavy Chain | 84 | DYGMH | 85 | YISSGSSTIYYADTV KG | 86 | NSRGFAY |
| | Light Chain | 87 | SASSSVSY MY | 88 | DTSNLAS | 89 | QQWSSYPRT |
| 47E12D12 -2 | Heavy Chain | 84 | DYGMH | 85 | YISSGSSTIYYADTV KG | 86 | NSRGFAY |
| | Light Chain | 90 | SASSSVSSM H | 91 | DTSKLAS | 92 | QQWNSYHLT |
| 48B6D7 | Heavy Chain | 93 | IVYWMQ | 94 | MILPNSDITNYNENEN FQT | 95 | QARYSAMD Y |
| | Light Chain | 96 | SASQGISNY LN | 97 | YTSNLHS | 98 | QHYSKFPYT |
| 45E4D7 | Heavy Chain | 99 | SHWIT | 100 | DIYPISGSTNNNEKF RN | 101 | IITVGGAYV MDY |
| | Light Chain | 75 | RASQSVRN - NLH | 76 | YVSQSIS | 77 | QQSNSWPLT |
| 27E10C5 | Heavy Chain | 72 | SQWMN | 73 | QIYPGNGDTNYNG KFKG | 74 | WFAY |
| | Light Chain | 102 | KSSQSLLNS SHQKNYLA | 103 | FASTRES | 104 | QQHYSTPWT |

### Example 14. Construction of mouse hybridoma-derived anti-DLL3 chimeric antibodies and their transient transfection expression in eukaryotic cells

The gene fragment of interest generated after splicing the sequenced heavy and light chain variable region of the monoclonal antibody of the present disclosure with the IgG1 heavy chain constant region and kappa light chain constant region was cloned into the pTT5 expression vector to prepare a transfection-grade expression plasmid.

Expi293F^{™} cells (Thermo Fisher Scientific) were cultured in a serum-free medium, seeded in a shake flask (Corning Inc.), and cultured in a 37°C, 8% CO₂ shaker. After adjusting the cell density, the recombinant expression vector containing the gene fragment of interest and PEI transfection reagent were mixed in an appropriate ratio and added into a cell culture flask. After 6 days of cell culture, the expression supernatant was collected, centrifuged at high speed to remove cell debris, and subjected to affinity purification using a Protein A column. The column was rinsed with PBS until the A280 reading dropped to baseline. The protein of interest was eluted with an acidic eluent at pH 3.0-pH 3.5 and neutralized with 1M Tris-HCl, pH 8.0-9.0. After the eluted sample was appropriately concentrated, the solution was changed to PBS and aliquoted for later use. Final purified chimeric antibodies were subjected to SDS-PAGE and HPLC purity analysis and A280 concentration determination.

### Example 15. In vitro, cell binding verification of mouse hybridoma-derived anti-DLL3 chimeric antibodies

SHP-77 and HEK293-cyno DLL3 cells were cultured. The culture medium for SHP-77 cells were RPMI1640+10% FBS, and the culture medium for HEK293 cyno DLL3 cells was DMEM+10% FBS+200 µg/ml Hygromycin. The cells were cultured in a T75 cell culture flask at a 37°C 5% CO₂ incubator. When using cells, they are washed with sterile DPBS, digested with 0.25% trypsin EDTA for about 5 minutes, and then stopped with a complete medium.

The digested cells were centrifuged at 1000 rpm for 5 min at room temperature. The supernatant was discarded and the cells were resuspended in 100 µL of 1% BSA (in PBS). The cells were counted and adjusted to a cell density of 1E6/m. The dilute cells were seeded in a 96-well round-bottom culture plate (corning 3799), and centrifuged at 1500 rpm for 5 min at 4°C. The supernatant was discarded and the cells were stored at 4°C until use. Antibody samples to be tested were diluted with 1% BSA (in PBS) at a starting concentration of 100 nM, downwards by a 10-fold gradient for 7 concentrations. The cells were resuspended with the diluted antibody at 100 µL/well and incubated for 1 hour at 4°C. The cells were centrifuged at 1500 rpm for 5 minutes at 4°C and the supernatant was discarded. The cells were resuspended and washed with 160 µL of 1%BSA (in PBS), centrifuged at 1500 rpm for 5 minutes at 4°C. The supernatant was discarded. The secondary antibody (goat anti human IgG Fc PE) was diluted 1: 200 with 1% BSA (in PBS) according to the manufacturer's instructions and the cells were resuspended with the diluted secondary antibody, 100 µL/well, and incubated for 0.5 h at 4°C. The cells were centrifuged at 1500 rpm for 5 minutes at 4°C and the supernatant was discarded. The cells were resuspended and washed with 160 µL of 1%BSA (in PBS), centrifuged at 1500 rpm for 5 minutes at 4°C. The supernatant was discarded. The cells were resuspended in 100 µL of 1% BSA (in PBS), and filtered across a 300 mesh gauze. The mean fluorescence intensity of the PE channels was measured by flow cytometry.

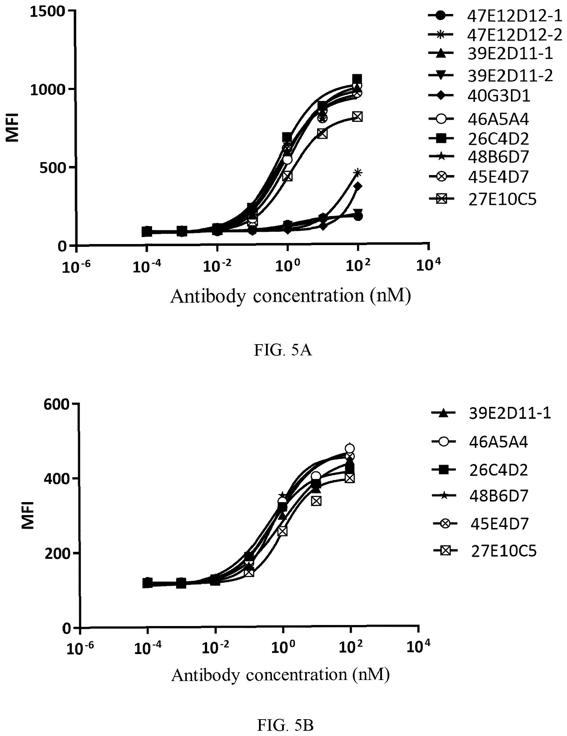

The FCS file was exported from the flow cytometer. The mean fluorescence intensity of PE channel (hereinafter referred to as MFI) of each sample was analyzed with the Flowjo software. The analyzed mean fluorescence intensity was imported into Graphpad to analyze the median-binding concentration (hereinafter referred to as EC50) of antibody and cell and the top mean fluorescence intensity (Top MFI). The results are shown in Table 11 and Figure 5.

**Table 11 Binding of mouse hybridoma-derived anti-DLL3 chimeric antibodies to DLL3-expressing cells**

| Clone No. | SHP-77 | | HEK293 - cyno DLL3 | |
|---|---|---|---|---|
| | EC₅₀ (nM) | Top Mean Fluorescence Intensity (Top MFI)) | EC₅₀ (nM) | Top Mean Fluorescence Intensity (Top MFI)) |
| 26C4D2 | 0.60 | 1053 | 0.43 | 421 |
| 39E2D11-1 | 0.85 | 999 | 1.03 | 445 |
| 39E2D11-2 | 8.63 | 193 | not detected | not detected |
| 40G3D1 | Unfitted | 369 | not detected | not detected |
| 46A5A4 | 1.18 | 1013 | 0.67 | 475 |
| 47E12D12-1 | 1.45 | 178 | not detected | not detected |
| 47E12D12-2 | 69.28 | 455 | not detected | not detected |
| 48B6D7 | 0.65 | 990 | 0.53 | 480 |
| 45E4D7 | 0.68 | 965 | 0.71 | 454 |
| 27E10C5 | 1.20 | 813 | 1.39 | 396 |

### Example 16. Humanization of mouse hybridoma-derived anti-DLL3 antibodies

10 chimeric antibodies were subjected to an expression purification test and cell-level binding test, and 3 clones were further selected for humanization design.

Humanization of murine anti-human DLL3 monoclonal antibodies was performed as disclosed in many references in the art. Briefly, a human constant domain was used in place of a parental (murine antibody) constant domain, and the human antibody sequence was selected based on the homology between murine and human antibodies. Based on the obtained VH/VL CDR typical structure of a murine antibody, the heavy and light chain variable region sequences were compared with the human antibody germline database to obtain human germline templates with high homology.

The CDR region of the murine antibody was grafted onto a selected corresponding humanized template to replace the humanized variable region and recombined with the IgG constant region (preferably IgG1 for heavy chain and kappa for light chain). Then, based on the three-dimensional structure of mouse-derived antibodies, reverse mutations were performed on embedded residues, residues that directly interact with the CDR region, and residues that have a significant impact on the conformation of VL and VH. Antibodies were designed by combining the following humanized light and heavy chain variable region sequences, as shown in Table 12.

**Table 12 Amino acid sequences of three mouse hybridomas-derived humanized antibody variable regions**

| Clone No. | | Variable Heavy Chain (VH) | Variable Light Chain (VL) |
|---|---|---|---|
| H1-39E2D11 | SEQ ID NO | 46 | 47 |
| | Amino | QVQLVQSGAEVKKPGASVKVS | EIVLTQSPATLSLSPGERATL |
| | Acid Sequence | CKASGYTFISYWITWVRQAPG QGLEWMGDIYPGSGSTTNYNE KFKSRVTMTRDTSTSTVYMELS SLRSEDTAVYYCARETTVGGAY AMDYWGQGTLVTVSS | SCRASQSINNNLHWYQQK PGQAPRLLIYYVSQSISGIP ARFSGSGSGTDFTLTISSLE PEDFAVYYCQQTNSWPLTF GGGTKLEIK |
| H2-39E2D11 | SEQ ID NO | 46 | 48 |
| | Amino Acid Sequence | QVQLVQSGAEVKKPGASVKVS CKASGYTFISYWITWVRQAPG QGLEWMGDIYPGSGSTTNYNE KFKSRVTMTRDTSTSTVYMELS SLRSEDTAVYYCARETTVGGAY AMDYWGQGTLVTVSS | EIVLTQSPATLSLSPGERATL SCRASQSINNNLHWYQQK PGQAPRLLIKYVSQSISGIP ARFSGSGSGTDFTLTISSLE PEDFAVYYCQQTNSWPLTF GGGTKLEIK |
| H-46A5A4 | SEQ ID NO | 49 | 50 |
| | Amino Acid Sequence | QVQLVQSGAEVKKPGASVKVS CKASGYTFTDHTIHWVRQAPG QGLEWMGYIYPRDGYTMYNE KFKGRVTMTRDTSTSTVYMEL SSLRSEDTAVYYCARAFHALDY WGQGTLVTVSS | DIQMTQSPSSLSASVGDRV TITCSASQGISNYLNWYQQ KPGKAPKLLIYYTSSLHSG VPSRFSGSGSGTDFTFTISS LQPEDIATYYCQQYSKLPY TFGGGTKLEIK |
| H1-45E4D7 | SEQ ID NO | 51 | 52 |
| | Amino Acid Sequence | QVQLVQSGAEVKKPGASVKVS CKASGYTFTSHWITWVRQAPG QGLEWMGDIYPISGSTNNNEKF RNRVTMTRDTSTSTVYMELSSL RSEDTAVYYCAKIITVGGAYVM | EIVMTQSPATLSVSPGERAT LSCRASQSVRNNLHWYQQ KPGQAPRLLIYYVSQSISGI PARFSGSGSGTEFTLTISSL QSEDFAVYYCQQSNSWPLT |
| | | DYWGQGTLVTVSS | FGGGTKLEIK |
| H2-45E4D7 | SEQ ID NO | 51 | 53 |
| | Amino Acid Sequence | QVQLVQSGAEVKKPGASVKVS CKASGYTFTSHWITWVRQAPG QGLEWMGDIYPISGSTNNNEKF RNRVTMTRDTSTSTVYMELSSL RSEDTAVYYCAKIITVGGAYVM DYWGQGTLVTVSS | EIVMTQSPATLSVSPGERAT LSCRASQSVRNNLHWYQQ KPGQAPRLLIKYVSQSISGI PARFSGSGSGTEFTLTISSL QSEDFAVYYCQQSNSWPLT FGGGTKLEIK |

### Example 17. In vitro, cell binding verification of mouse hybridoma-derived humanized anti-DLL3 antibodies

SHP-77 cells were cultured. The culture medium for SHP-77 cells was RPMI1640+10% FBS. The cells were cultured in a T75 cell culture flask at a 37°C 5% CO₂ incubator. When using cells, they are washed with sterile DPBS, digested with 0.25% trypsin EDTA for about 5 minutes, and then stopped with a complete medium.

The digested cells were centrifuged at 1000 rpm for 5 min at room temperature. The supernatant was discarded and the cells were resuspended in 100 µL of 1% BSA (in PBS). The cells were counted and adjusted to a cell density of 1E6/m. The dilute cells were plated in a 96-well round-bottom culture plate (corning 3799), and centrifuged at 1500 rpm for 5 min at 4°C. The supernatant was discarded and the cells were stored at 4°C until use. Antibody samples to be tested were diluted with 1% BSA (in PBS) at a starting concentration of 100 nM, downwards by a 10-fold gradient for 7 concentrations. The cells were resuspended with the diluted antibody at 100 µL/well and incubated for 1 hour at 4°C. The cells were centrifuged at 1500 rpm for 5 minutes at 4°C and the supernatant was discarded. The cells were resuspended and washed with 160 µL of 1%BSA (in PBS), centrifuged at 1500 rpm for 5 minutes at 4°C. The supernatant was discarded. The secondary antibody (goat anti human IgG Fc PE) was diluted 1: 200 with 1% BSA (in PBS) according to the manufacturer's instructions and the cells were resuspended with the diluted secondary antibody, 100 µL/well, and incubated for 0.5 h at 4°C. The cells were centrifuged at 1500 rpm for 5 minutes at 4°C and the supernatant was discarded. The cells were resuspended and washed with 160 µL of 1%BSA (in PBS), centrifuged at 1500 rpm for 5 minutes at 4°C. The supernatant was discarded. The cells were resuspended in 100 µL of 1% BSA (in PBS), and filtered across a 300 mesh gauze. The mean fluorescence intensity of the PE channels was measured by flow cytometry.

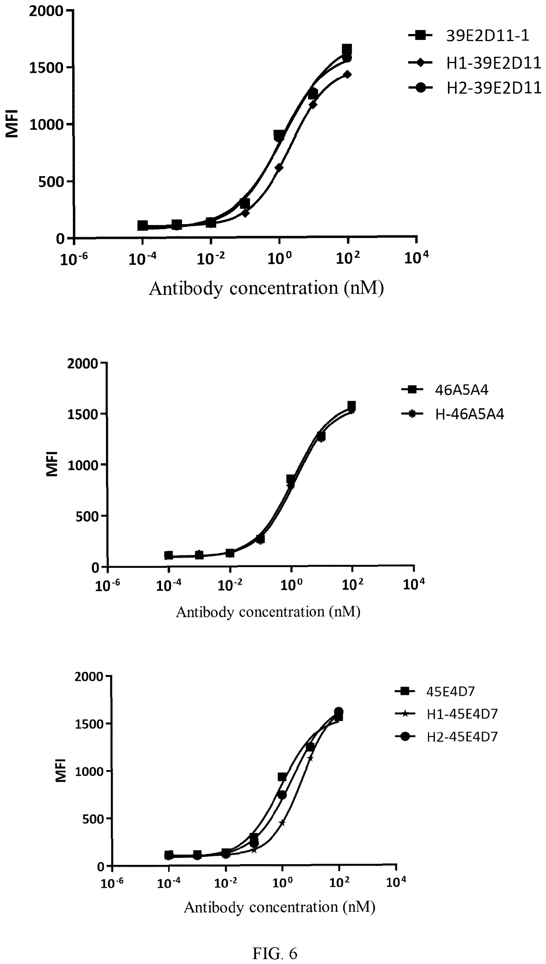

The FCS file was exported from the flow cytometer. The mean fluorescence intensity of the PE channel (hereinafter referred to as MFI) of each sample was analyzed with the Flowjo software. The analyzed mean fluorescence intensity was imported into Graphpad to analyze the median-binding concentration (hereinafter referred to as EC50) of antibody and cell and the top mean fluorescence intensity (Top MFI). The results are shown in Table 13 and Figure 6.

**Table 13. Binding of mouse hybridoma-derived humanized anti-DLL3 antibodies to DLL3-expressing cells (SHP-77)**

| Clone No. | SHP-77 | |
|---|---|---|
| | EC₅₀ (nM) | Top Mean Fluorescence Intensity (Top MFI)) |
| 39E2D11-1 | 1.491 | 1647 |
| H1-39E2D11 | 2.128 | 1426 |
| H2-39E2D11 | 1.125 | 1574 |
| 46A5A4 | 1.204 | 1574 |
| H-46A5A4 | 1.348 | 1525 |
| 45E4D7 | 0.894 | 1561 |
| H1-45E4D7 | 5.163 | 1588 |
| H2-45E4D7 | 2.228 | 1614 |

### Example 18. Biacore affinity experiment of hybridoma-derived humanized anti-d113 antibodies

The affinity and kinetic properties of the humanized anti-DLL3 antibody to human DLL3 were analyzed using a Biacore 8K instrument. CM5 chips were activated with EDC and NHS, followed by immobilization of anti-human Fc murine mAb and blocked with ethanolamine.

To determine the affinity to human DLL3 and kinetic properties, the DLL3 humanized antibody was diluted to 0.2 µg/mL with HBS-EP + (10 mM HEPES, pH 7.4, 150 mM NaCl, 3 mM EDTA, 0.05% P20) buffer and captured at a flow rate of 10 µL/min for 45 s. Human DLL3 was diluted two-fold serially to serial concentrations (100 nM-0.39 nM) and associated for 90 s and dissociated for 600 s at a flow rate of 50 µL/min.

After completion of each round of the experiment, the captured antibody was removed together with the antigen by washing with a 3M MgCl₂ solution at a flow rate of 30 µL/min for 30 s to complete the regeneration of the chip. The raw data were analyzed using Biacore Insight Evaluation Software (3.0.12.15655) software and fitted with a (1: 1) Langmuir model with. The results were shown in Table 14.

**Table 14. Affinity test results of hybridoma-derived humanized anti-DLL3 antibodies with human DLL3 antigen proteins**

| Clone No. | ka (1/Ms) | kd(1/s) | KD(M) |
|---|---|---|---|
| H2-39E2D11 | 3.13E+05 | 1.80E-04 | 5.76E-10 |
| H-46A5A4 | 2.19E+05 | 1.78E-04 | 8.11E-10 |
| H2-45E4D7 | 2.35E+05 | 5.78E-05 | 2.46E-10 |

### Example 19. Binding experiments of hybridoma-derived humanized anti-DLL3 antibodies with the family proteins DLL1 and DLL4

The antigenic proteins human DLL1 (SinoBiological, cat. No. 11635-H08H) or human DLL4 (SinoBiological, cat. No. 10171-H08H) were dissolved in 1 × PBS at a concentration of 1 µg/mL. The antigen was then added to a high-affinity ELISA plate (Biolegend, cat. No. 423501) at 100 µL/well and left overnight at 4°C. The antigen was washed three times with PBST, 300 µL/well. The antigen was blocked with 1% BSA (in PBST), 200 µL/well, and incubated at 37°C for 1.5 hours.

Antibodies to be tested were diluted with 1% BSA (in PBS) at a starting concentration of 100 nM, downwards by a 10-fold gradient for 7 concentrations. The plates were washed three times with PBST, 300 µL/well. The diluted antibody was added to an ELISA plate at 100 µL/well and incubated at room temperature for 2 hours. The antibodies were washed three times with PBST, 300 µL/well. The secondary antibodies (goat anti-human IgG Fc for DLL4 (HRP), 1: 20000 dilution; HRP goat anti-mouse IgG (H + L) for DLL1, 1: 10000 dilution) were diluted with 1% BSA in PBST. The diluted secondary antibodies were added to the ELISA plate at 100 µL/well and incubated at room temperature for 1 hour. The plates were washed 6 times with PBST, 300 µL/well. The developing solution (TMA and TMB 1:1 mixed) was prepared. The developing solution was added to the plate, 100 µL/well, and incubated in the dark for 5 min. 50 µL of ELISA stop solution was added to the plate and shaken well.

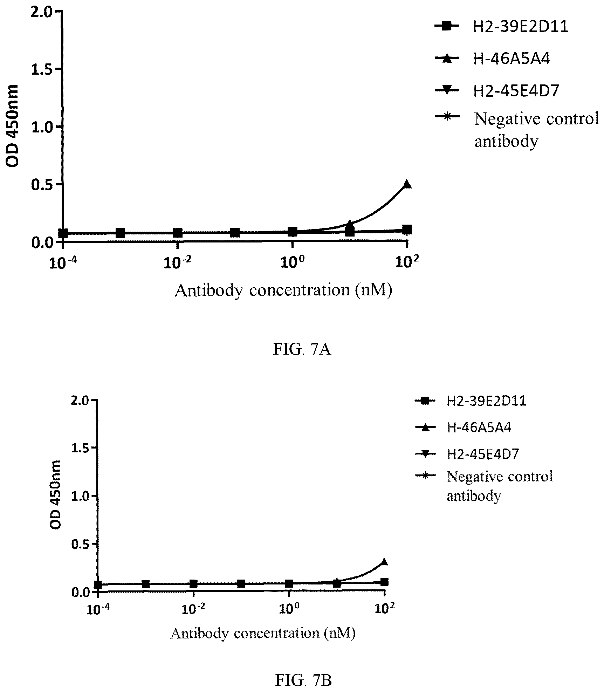

OD450s were read on the envision and plotted on the GraphPad for EC₅₀ values. The results are shown in FIG. 7, which show that the antibodies tested did not non-specifically bind to either of the family proteins DLL1 and DLL4.

### Example 20. Preparation of hybridoma-derived humanized anti-DLL3 antibody variants

After post-translational modification (PTM) analysis of the antibodies of the present disclosure, it was found that there was one deamidation site in the light chain variable region of H2-39E2D11, and site-directed mutation for a single site was performed on amino acid 99 to prepare three mutants of H2-39E2D11: H2-39E2D11-NA, H2-39E2D11-QS, and H2-39E2D11-AS, respectively. The variable region amino acid sequences of the three variants are shown in Table 15.

**Table 15 Amino acid sequences of H2-39E2D11 humanized antibody**

| Clone No. | | Variable Heavy Chain (VH) | Variable Light Chain (VL) | LCDR3 |
|---|---|---|---|---|
| H2-39E2D11 -NA | SEQ ID NO | 46 | 54 | 112 |
| | Amino Acid Sequence | QVQLVQSGAEVKKPGASVK VSCKASGYTFISYWITWVRQ APGQGLEWMGDIYPGSGST TNYNEKFKSRVTMTRDTSTS TVYMELSSLRSEDTAVYYCA RETTVGGAYAMDYWGQGT LVTVSS | EIVLTQSPATLSLSPGERA TLSCRASQSINNNLHWY QQKPGQAPRLLIKYVSQ SISGIPARFSGSGSGTDFT LTISSLEPEDFAVYYCQQ TNAWPLTFGGGTKLEIK | QQTNAWPL T |
| H2-39E2D11 -QS | SEQ ID NO | 46 | 55 | 113 |
| | Amino Acid Sequence | QVQLVQSGAEVKKPGASVK VSCKASGYTFISYWITWVRQ APGQGLEWMGDIYPGSGST TNYNEKFKSRVTMTRDTSTS TVYMELSSLRSEDTAVYYCA RETTVGGAYAMDYWGQGT LVTVSS | EIVLTQSPATLSLSPGERA TLSCRASQSINNNLHWY QQKPGQAPRLLIKYVSQ SISGIPARFSGSGSGTDFT LTISSLEPEDFAVYYCQQ TQSWPLTFGGGTKLEIK | QQTQSWPL T |
| H2-39E2D11 -AS | SEQ ID NO | 46 | 56 | 114 |
| | Amino Acid Sequence | QVQLVQSGAEVKKPGASVK VSCKASGYTFISYWITWVRQ APGQGLEWMGDIYPGSGST TNYNEKFKSRVTMTRDTSTS TVYMELSSLRSEDTAVYYCA RETTVGGAYAMDYWGQGT | EIVLTQSPATLSLSPGERA TLSCRASQSINNNLHWY QQKPGQAPRLLIKYVSQ SISGIPARFSGSGSGTDFT LTISSLEPEDFAVYYCQQ TASWPLTFGGGTKLEIK | QQTASWPL T |
| | | LVTVSS | | |

The three variants were prepared as described in Example 5. After transient expression in mammalian cell lines, affinity assays were performed using SHP-77 cells. The results shown in Table 8 show that the variants were able to eliminate the risk of post-translational modification without significant changes in binding at the cellular level. Affinity assay results using human DLL3 antigen proteins are shown in Table 16.

**Table 16A Affinity test results of H2-39E2D11 humanized antibody variant with human DLL3 antigen protein**

| Clone No. | ka (1/Ms) | kd(1/s) | KD(M) |
|---|---|---|---|
| H2-39E2D11 | 1.81E+05 | 1.34E-04 | 7.39E-10 |
| H2-39E2D11-NA | 2.47E+05 | 1.20E-04 | 4.84E-10 |
| H2-39E2D11-QS | 1.71E+05 | 3.91E-05 | 2.29E-10 |
| H2-39E2D11-AS | 2.06E+05 | 3.62E-04 | 1.76E-09 |

**Table 16B Affinity test results of H2-39E2D11 humanized antibody variant with Cynomolgus monkey DLL3 antigen protein**

| Clone No. | ka (1/Ms) | kd(1/s) | KD(M) |
|---|---|---|---|
| H2-39E2D11 | 2.21E+05 | 3.16E-05 | 1.43E-10 |
| H2-39E2D11-NA | 2.33E+05 | 8.09E-05 | 3.46E-10 |
| H2-39E2D11-QS | 1.98E+05 | 8.59E-05 | 4.34E-10 |
| H2-39E2D11-AS | 2.62E+05 | 4.27E-04 | 1.63E-09 |

The embodiments of the present disclosure described above are merely exemplary, and any person skilled in the art will recognize, or determine the equivalence of numerous specific compounds, materials, and operations without the need for unconventional experiments. All of these equivalents are within the scope of the present disclosure and are included in the claims.

## Claims

1. A DLL3 binding molecule or an antigen binding fragment thereof, comprising a heavy chain variable region (VH) , wherein the heavy chain variable region comprises HCDR1, HCDR2, and HCDR3, the amino acid sequences of the HCDR1, HCDR2, and HCDR3 are respectively:
a) SEQ ID NOs: 6, 7, and 8; or
b) SEQ ID NOs: 9, 10, and 11; or
c) SEQ ID NOs: 12, 13, and 14; or
d) SEQ ID NOs: 15, 16, and 17; or
e) SEQ ID NOs: 18, 19, and 20; or
f) SEQ ID NOs: 6, 7, and 110; or
g) SEQ ID NOs: 6, 7, and 111;
preferably, the heavy chain variable region comprises an amino acid sequence selected from any one of SEQ ID NOs: 1-5, 21-29, or comprises an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to any one of SEQ ID NOs: 1-5, 21-29.

2. A DLL3 binding molecule or antigen binding fragment thereof, comprising a heavy chain variable region (VH), wherein the heavy chain variable region comprises HCDR1, HCDR2, and HCDR3 and a light chain variable region (VL) , wherein the light chain variable region comprises LCDR1, LCDR2, and LCDR3, the amino acid sequences of the HCDR1, HCDR2, HCDR3, LCDR1, LCDR2, and LCDR3 are respectively:
a) SEQ ID NOs: 57, 58, 59, 60, 61, and 62; or
b) SEQ ID NOs: 63, 64, 65, 66, 67, and 68; or
c) SEQ ID NOs: 69, 70, 71, 66, 67, and 68; or
d) SEQ ID NOs: 72, 73, 74, 75, 76 and 77; or
e) SEQ ID NOs: 78, 79, 80, 81, 82, and 83; or
f) SEQ ID NOs: 84, 85, 86, 87, 88, and 89; or
g) SEQ ID NOs: 84, 85, 86, 90, 91 and 92; or
h) SEQ ID NOs: 93, 94, 95, 96, 97, and 98; or
i) SEQ ID NOs: 99, 100, 101, 75, 76 and 77; or
j) SEQ ID NOs: 72, 73, 74, 102, 103 and 104; or
k) SEQ ID NOs: 63, 64, 65, 66, 67, and 112; or
l) SEQ ID NOs: 63, 64, 65, 66, 67, and 113; or
m) SEQ ID NOs: 63, 64, 65, 66, 67, and 114; or
preferably, the heavy chain variable region comprises an amino acid sequence selected from any one of SEQ ID NOs: 30, 32, 34, 35, 37, 39, 42, 44, 46, 49, or 51, or comprises an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to any one of SEQ ID NOs: 30, 32, 34, 35, 37, 39, 42, 44, 46, 49, or 51; and/or
the light chain variable region comprises an amino acid sequence selected from any one of SEQ ID NOs: 31, 33, 36, 38, 40, 41, 43, 45, 47, 48, 50, 52, 53, 54, 55, or 56, or comprises an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to any one of SEQ ID NOs: 31, 33, 36, 38, 40, 41, 43, 45, 47, 48, 50, 52, 53, 54, 55, or 56;
more preferably, the heavy chain variable region and the light chain variable region each comprise sequences selected from a group consisting of:
1) SEQ ID NOs: 30 and 31; or
2) SEQ ID NOs: 32 and 33; or
3) SEQ ID NOs: 34 and 33; or
4) SEQ ID NOs: 35 and 36; or
5) SEQ ID NOs: 37 and 38; or
6) SEQ ID NOs: 39 and 40; or
7) SEQ ID NOs: 39 and 41; or
8) SEQ ID NOs: 42 and 43; or
9) SEQ ID NOs: 44 and 36; or
10) SEQ ID NOs: 35 and 45; or
11) SEQ ID NOs: 46 and 47; or
12) SEQ ID NOs: 46 and 48; or
13) SEQ ID NOs: 49 and 50; or
14) SEQ ID NOs: 51 and 52; or
15) SEQ ID NOs: 51 and 53; or
16) SEQ ID NOs: 46 and 54; or
17) SEQ ID NOs: 46 and 55; or
18) SEQ ID NOs: 46 and 56.

3. The DLL3 binding molecule or the antigen binding fragment thereof according to any one of the preceding claims further having one or more of the following characteristics:
i) further comprising a heavy chain constant region and/or a light chain constant region; preferably, the heavy chain constant region comprises an Fc; more preferably, Fc is derived from murine or human; more preferably, the sequence of the Fc is native or modified;
ii) the DLL3 binding molecule or the antigen binding fragment thereof is a monoclonal antibody, a bispecific binding molecule, a multispecific binding molecule, a humanized antibody, a chimeric antibody, a modified antibody, a fully human antibody, a full-length antibody, a heavy chain antibody, a nanobody, an Fab, an Fv, an scFv, an F(ab')₂, a linear antibody, or a single domain antibody; and/or
iii) in the form of IgG1, IgG2, IgG3 or IgG4.

4. A conjugate formed by coupling the DLL3 binding molecule or the antigen binding fragment thereof according to any one of the preceding claims to a capture label or a detection label; preferably, the detection label comprises a radionuclide, a luminescent substance, a colored substance, or an enzyme.

5. An antibody drug conjugate formed by coupling the DLL3 binding molecule or the antigen binding fragment thereof according to any one of the preceding claims with another biologically active molecule; preferably, the other biologically active molecule is a small molecule drug; preferably, the DLL3 binding molecule or the antigen binding fragment thereof is linked to the other biologically active molecule via a linker.

6. A nucleic acid encoding the DLL3 binding molecule or the antigen binding fragment thereof according to any one of the preceding claims, or a recombinant vector comprising the nucleic acid, or a host cell comprising the nucleic acid or the recombinant vector; preferably, the host cell is a prokaryotic cell, preferably E. coli, or a eukaryotic cell, preferably a mammalian cell or yeast; further preferably, the mammalian cell is a CHO cell or a HEK293 cell.

7. A method for preparing the DLL3 binding molecule or the antigen binding fragment thereof according to any one of the preceding claims, the method comprising culturing the host cell according to claim 6 under suitable conditions and purifying the expression product from the cell.

8. Use of the DLL3 binding molecule or the antigen binding fragment thereof according to any one of the preceding claims in the manufacture of a medicament for the treatment or amelioration of a tumor;
preferably, the drug targets tumor cells aberrantly expressing DLL3; preferably, the tumor is selected from small cell lung cancer, glioblastoma, neuroendocrine cancer, melanoma, pancreatic cancer, rectal cancer, and metastatic cancers of the aforementioned tumors.

9. Use of the DLL3 binding molecule or the antigen binding fragment thereof according to any one of the preceding claims in the manufacture of a detection or diagnostic reagent; preferably, the detection reagent is used for detecting expression of DLL3; the diagnostic reagent is used for diagnosing a tumor; preferably, the tumor is selected from small cell lung cancer, glioblastoma, neuroendocrine cancer, melanoma, pancreatic cancer, rectal cancer, and metastatic cancers of the aforementioned tumors.

10. A method for detecting DLL3 expression in a sample, comprising:
(1) contacting the sample with the DLL3 binding molecule or the antigen binding fragment thereof according to any one of the preceding claims;
(2) detecting the formation of a complex of the DLL3 binding molecule or the antigen binding fragment thereof and DLL3; optionally, the DLL3 binding molecule or the antigen binding fragment thereof is detectably labeled.

11. A pharmaceutical composition comprising an effective amount of the DLL3 binding molecule or the antigen binding fragment thereof according to any one of the preceding claims, an effective amount of the antibody drug conjugate according to claim 5, or an effective amount of the nucleic acid or recombinant vector or host cell according to claim 6;
preferably, it further comprises a pharmaceutically acceptable carrier; preferably, it further comprises one or more additional therapeutic agents.

12. A drug box or kit comprising a container and the pharmaceutical composition according to claim 11 in the container.

13. A method for inducing death of a cell expressing DLL3, the method comprising contacting the cell with the pharmaceutical composition according to claim 11, wherein the cell expressing DLL3 is a tumor cell;
preferably, the tumor cell is a cell selected from the following tumors: small cell lung cancer, glioblastoma, neuroendocrine cancer, melanoma, pancreatic cancer, rectal cancer, and metastatic cancers of the aforementioned tumors.

14. A method for treating a disease related to DLL3 expression in a subject, comprising administering the pharmaceutical composition according to claim 11 and/or the drug box or kit according to claim 12 to the subject in need;
preferably, the disease is a tumor; small cell lung cancer, glioblastoma, neuroendocrine cancer, melanoma, pancreatic cancer, rectal cancer, and metastatic cancers of the aforementioned tumors;
more preferably, the method further comprises administering an additional therapeutic agent to the subject.
